(19) Europäisches Patentamt
European Patent Office
Office européen des brevets

(11) Veröffentlichungsnummer: 0 351 662
A2

(12) # EUROPÄISCHE PATENTANMELDUNG

(21) Anmeldenummer: 89112403.4

(22) Anmeldetag: 07.07.89

(51) Int. Cl.4: C07D 277/58 , A01N 43/80

(30) Priorität: 20.07.88 DE 3824520
21.12.88 DE 3842970

(43) Veröffentlichungstag der Anmeldung:
24.01.90 Patentblatt 90/04

(84) Benannte Vertragsstaaten:
BE CH DE ES FR GB GR IT LI NL

(71) Anmelder: BAYER AG

D-5090 Leverkusen 1 Bayerwerk(DE)

(72) Erfinder: Beck, Gunther, Dr.
Am Mittelberg 19
D-5090 Leverkusen 1(DE)
Erfinder: Brandes, Wilhelm, Dr.
Eichendorffstrasse 3
D-5653 Leichlingen 1(DE)
Erfinder: Dutzmann, Stefan, Dr.
Leinenweberweg 33
D-4000 Düsseldorf 13(DE)
Erfinder: Paulus, Wilfried, Dr.
Deswatinesstrasse 90
D-4150 Krefeld 1(DE)

(54) **4-Halogen-5-nitrothiazol-Derivate, Verfahren zur ihrer Herstellung, ihre Verwendung als Schädlingsbekämpfungsmittel und neue Zwischenprodukte.**

(57) 4-Halogen-5-nitrothiazol-Derivate der Formel (I)

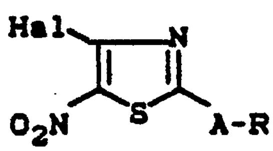

(I)

in welcher
Hal, A und R die in der Beschreibung gegebenen Bedeutungen haben, mehrere Verfahren zu ihrer Herstellung und ihre Verwendung zur Bekämpfung von Schadorganismen, vor allem von phytopathogenen Pilzen und neue Zwischenprodukte.

Die neuen 4-Halogen-5-nitrothiazol-Derivate sind durch die Formel (I) allgemein definiert. Man kann sie nach Analogieverfahren herstellen, z. B. indem man ein geeignetes 2,4-Dihalogen-5-nitrothiazol mit einem geeigneten Nucleophil oder mit einem geeigneten Dimethylarylamin umsetzt oder indem man ein geeignetes, erfindungsgemäßes 4-Halogen-5-nitrothiazolylsulfid mit mindestens 2 Mol bzw. mit 1 Mol eines Oxidationsmittels umsetzt.

Einige 2,4-Dihalogen-5-nitrothiazole der Formel (II) sind auch neu und können aus 2,4-Dichlor-5-nitrothiazol z. B. mit geeigneten Metalliodiden hergestellt werden.

EP 0 351 662 A2

**4-Halogen-5-nitrothiazol-Derivate, Verfahren zur ihrer Herstellung, ihre Verwendung als Schädlingsbekämpfungsmittel und neue Zwischenprodukte**

Die vorliegende Erfindung betrifft neue 4-Halogen-5-nitrothiazol-Derivate, mehrere Verfahren zu ihrer Herstellung sowie ihre Verwendung als Schädlingsbekämpfungsmitteln und einige neue Zwischenprodukte.

Es ist bekannt, daß Thiazole, die z. B. durch Hydroxyphenoxy substituiert sind, in der Medizin Verwendung finden zur Behandlung von Tumoren (vergl. WO 88/00944).

Weiterhin ist bekannt, daß 2,4-Dichlor-5-nitrothiazol eine mikrobizide, vor allem eine fungizide Wirkung im Pflanzenschutz aufweist (vergl. DE-OS 3 518 520).

Auch sind Thiazol-Derivate, wie z.B. 4,5-Dichlor-2-propinyloxy-thiazol, als Synergisten bekannt (vgl. DE-OS 3 030 661).

Weiterhin ist bekannt, daß bestimmte substituierte Thiazole, wie z.B. 4-Benzimidazol-2-yl-thiazol (Thiabendazol), als Fungizide zum Materialschutz verwendet werden (vgl. z.B. R. Wegler "Chemie der Pflanzenschutz-und Schädlingsbekämpfungsmittel", Bd. 2, S. 124 und Bd. 3, S. 292; Springer Verlag, Berlin, Heidelberg, N.Y. 1970).

Das Wirkungsspektrum dieser vorbekannten Verbindungen ist jedoch lückenhaft und ihre mikrobizide Wirksamkeit ist in bestimmten Indikationsgebieten nicht immer befriedigend.

Es wurden neue 4-Halogen-5-nitrothiazol-Derivate der Formel (I)

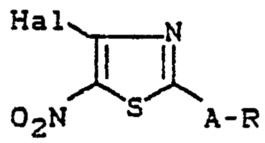

(I)

in welcher

Hal für Halogen steht,

A für O, S, SO, $SO_2$ oder N-$R^1$ steht,

worin

$R^1$ für Wasserstoff, Alkyl, Alkenyl, Halogenalkyl, Cyanalkyl oder für jeweils gegebenenfalls ein-bis mehrfach, gleich oder verschieden substituiertes Cycloalkyl, Aryl oder Aralkyl steht und

R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein-bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht oder

R und $R^1$ zusammen mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann,

mit der Ausnahme, daß R nicht Wasserstoff ist, wenn A für SO oder $SO_2$ steht,

gefunden.

Weiterhin wurde gefunden, daß man die 4-Halogen-5-nitrothiazol-Derivate der Formel (I)

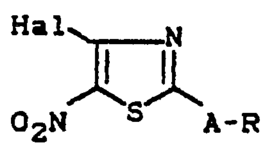

(I)

in welcher

Hal für Halogen steht,

A für O, S, SO, $SO_2$ oder N-$R^1$ steht,
worin
$R^1$ für Wasserstoff, Alkyl, Alkenyl, Halogenalkyl, Cyanalkyl oder für jeweils gegebenenfalls ein-bis mehrfach, gleich oder verschieden substituiertes Cycloalkyl, Aryl oder Aralkyl steht und
R für Wasserstoff, Alkyl, Alkenyl, Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht oder
R und $R^1$ zusammen mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann,
mit der Ausnahme, daß R nicht Wasserstoff ist, wenn A für SO oder $SO_2$ steht, erhält, wenn man

a) für den Fall, daß A für O, S, $SO_2$ oder $NR^1$ steht,
2,4-Dihalogen-5-nitrothiazole der Formel (II)

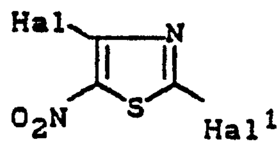

(II)

in welcher
Hal und $Hal^1$ gleiche oder verschiedene Halogenatome bedeuten,
mit Nucleophilen der Formel (III)
$H - A' - R$ (III)
in welcher
R die oben angegebene Bedeutung hat und
$A'$ für O, S, $SO_2$ oder $NR^1$ steht, wobei $R^1$ die vorher angegebene Bedeutung hat, oder deren Metallsalze gegebenenfalls in Gegenwart von Säurebindemitteln sowie in Gegenwart von Verdünnungsmitteln umsetzt, oder

b) für den Fall, daß A-R für

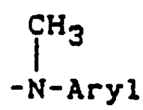

steht, 2,4-Dihalogen-5-nitrothiazole der Formel (II) mit N,N-Dimethylarylaminen der Formel (IV)

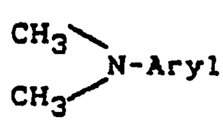

(IV)

in welcher Aryl die unter R für Aryl angegebene Bedeutung hat, in Gegenwart von Verdünnungsmitteln umsetzt,

c) für den Fall, daß A für $SO_2$ bzw. SO steht, 4-Halogen-5-nitrothiazolyl-sulfide der Formel (V)

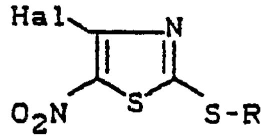

(V)

in welcher Hal und R die oben angegebene Bedeutung haben,

α) mit mindestens 2 Mol eines Oxidationsmittels in Gegenwart eines unter den Reaktionsbedingungen inerten Verdünnungsmittels umsetzt bzw.

β) mit etwa 1 Mol eines Oxidationsmittels in Gegenwart eines unter den Reaktionsbedingungen inerten Verdünnungsmittels umsetzt.

Schließlich wurde gefunden, daß die neuen 4-Halogen-5-nitrothiazol-Derivate der Formel (I) eine starke Wirkung gegen Schädlinge, vor allem gegen Pilze im Pflanzenreich und gegen Mikroben in technischen Materialien, haben.

Überraschenderweise zeigen die erfindungsgemäßen 4-Halogen-5-nitrothiazol-Derivate der Formel (I) u. a. eine erheblich höhere fungizide Wirksamkeit im Pflanzenschutz als z. B. die aus dem Stand der Technik bekannten Handelsprodukte Captan (N-Trichlormethylthio-tetrahydrophthalimid), das Euparen (N,N-Dimethyl-N′-phenyl-N′-(fluordichlormethylthio)-sulfamid oder das Curzate [2-Cyan-N-(ethylaminocarbonyl)-2-methoximino)-acetamid], welches wirkungsmäßig naheliegende Verbindungen sind; außerdem gegenüber dem bekannten 4-Benzimidazol-2-yl-thiazol eine bessere mikrobizide Wirkung im Materialschutz.

Im Rahmen der vorliegenden Erfindung haben die Substituenten die angegebenen Bedeutungen, im allgemeinen seien einige Substituenten im folgenden aufgezählt.

Halogen kann, überall wo nicht anders angegeben, Fluor, Chlor, Brom und Iod bedeuten.

Alkyl steht hierbei im allgemeinen in R für einen geradkettigen oder verzweigten Kohlenwasserstoffrest mit 1 bis 12 Kohlenstoffatomen. Genannt seien beispielsweise Methyl, Ethyl, Propyl, Isopropyl, Butyl, Isobutyl, t-Butyl, Pentyl, Isopentyl, Hexyl, Isohexyl, Heptyl, Isoheptyl, Octyl und Isooctyl.

Alkenyl steht im allgemeinen in R für einen geradkettigen oder verzweigten Kohlenwasserstoffrest mit 3 bis 12 Kohlenstoffatomen und einer oder mehreren, bevorzugt mit einer oder zwei Doppelbindungen. Beispielsweise seien Allyl, Propenyl, Isopropenyl, Butenyl, Isobutenyl, Pentenyl, Isopentenyl, Hexyl, Isohexyl, Heptenyl, Isoheptenyl, Octenyl, Isooctenyl genannt.

Cycloalkyl steht im allgemeinen in R für einen cyclischen Kohlenwasserstoffrest mit 3 bis 8 Kohlenstoffatomen. Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Halogenalkyl steht im allgemeinen für geradkettiges oder verzweigtes Niedrigalkyl mit 1 bis 8 Kohlenstoffatomen und einem bis mehreren Halogenatomen. Beispielhaft seien genannt: Fluormethyl, Chlormethyl, Brommethyl, Fluorethyl, Chlorethyl, Bromethyl, Fluorpropyl, Chlorpropyl, Brompropyl, Fluorbutyl, Chlorbutyl, Brombutyl, Fluorisopropyl, Chlorisopropyl, Bromisopropyl, Difluormethyl, Trifluormethyl, Dichlormethyl, Trichlormethyl, Difluorethyl, Dichlorethyl, Trifluorethyl, Tetrafluorethyl, Pentafluorethyl, Trichlorethyl, Trifluorpropyl. Ganz besonders bevorzugt sind Trifluormethyl, Difluormethyl, Fluormethyl, Chlormethyl und Trifluorethyl.

Halogenalkenyl steht im allgemeinen für geradkettiges oder verzweigtes Alkenyl mit bis zu 8 Kohlenstoffatomen und einem bis mehreren, gleichen oder verschiedenen Halogenatomen und mit einer oder mehreren Doppelbindungen. Bevorzugt sind Reste mit einer Doppelbindung. Beispielhaft seien genannt: 2,2-Dichlorvinyl, 1,2,2-Trichlorvinyl.

Aryl kann für einen aromatischen Kohlenwasserstoffrest mit 6 bis 12 Kohlenstoffatomen stehen. Beispielsweise seien Phenyl oder Naphthyl genannt. Bevorzugt sind Phenyl und Naphthyl.

Aralkyl kann für einen Rest mit 7 bis 16 Kohlenstoffatomen stehen, wobei ein geradkettiger oder verzweigter Alkylrest mit 1 bis 4 Kohlenstoffatomen durch einen aromatischen Rest mit 6 bis 12 Kohlenstoffatomen substituiert sein kann. Beispielsweise seien Benzyl, Phenylethyl und Phenylpropyl genannt. Bevorzugt sind Benzyl und Phenylethyl.

Die Aryl- und Aralkylreste können gegebenenfalls einbis mehrfach, gleich oder verschieden substituiert sein.

Alkoxyalkyl bzw. Alkylmercaptoalkyl stehen im allgemeinen für einen über Sauerstoff bzw. Schwefel gebundenen, geradkettigen oder verzweigten Kohlenwasserstoffrest mit 1 bis 4 Kohlenstoffatomen je Alkylteil. Beispielsweise seien genannt Methoxymethyl, Methoxyethyl, Methoxypropyl, Methoxybutyl, Ethoxymethyl, Ethoxyethyl, Ethoxypropyl, Ethoxybutyl, Propoxymethyl, Propoxyethyl, Propoxypropyl, Propoxybutyl, Butoxymethyl, Butoxyethyl, Butoxypropyl und Butoxybutyl.

Ferner Methylthiomethyl, Methylthioethyl, Methylthiopropyl, Methylthiobutyl, Ethylthiomethyl, Ethylthioethyl, Ethylthiopropyl, Ethylthiobutyl, Propylthiomethyl, Propylthioethyl, Propylthiopropyl, Propylthiobutyl, Butylthiomethyl, Butylthioethyl, Butylthiopropyl und Butylthiobutyl.

Die erfindungsgemäßen 4-Halogen-5-nitrothiazol-Derivate sind durch die Formel (I) allgemein definiert. Bevorzugt sind Verbindungen der Formel (I), in welcher
Hal für Fluor, Chlor, Brom oder Iod steht,
A für O, S, SO, $SO_2$ oder $NR^1$ steht, wobei
$R^1$ für Wasserstoff, Alkyl mit 1 bis 4 Kohlenstoffatomen, Alkenyl mit 3 bis 5 Kohlenstoffatomen, Halogenalkyl mit 1 bis 3 Kohlenstoffatomen und 1 bis 5 gleichen oder verschiedenen Halogenatomen, für Cyanalkyl mit 1 oder 2 Kohlenstoffatomen im Alkylteil, für Cycloalkyl mit 3 bis 6 Kohlenstoffatomen, für gegebenenfalls jeweils ein- bis dreifach, gleich oder verschieden durch Alkyl mit 1 bis 4 Kohlenstoffatomen, Fluor, Chlor, Brom oder Alkoxy mit 1 bis 4 Kohlenstoffatomen substituiertes Phenyl oder Phenylalkyl mit 1 bis 3 Kohlenstoffatomen im Alkylteil steht und
R für Wasserstoff, Alkyl mit 1 bis 12 Kohlenstoffatomen, Alkenyl mit 3 bis 12 Kohlenstoffatomen, Alkinyl mit 4 bis 12 Kohlenstoffatomen, Halogenalkyl, Halogenalkenyl oder Halogenalkinyl mit jeweils bis zu 8 Kohlenstoffatomen und 1 bis 10 gleichen oder verschiedenen Halogenatomen steht, für Alkoxyalkyl, Alkylmercaptoalkyl oder Cyanalkyl mit 1 bis 4 Kohlenstoffatomen je Alkylteil steht, für Phenyloxyalkyl oder Phenylmercaptoalkyl mit 1 bis 4 Kohlenstoffatomen im Alkylteil steht, wobei die Phenylreste gegebenenfalls ein- bis dreifach, gleich oder verschieden durch Fluor, Chlor, Brom oder Alkyl mit 1 bis 4 Kohlenstoffatomen substituiert sein können, für gegebenenfalls ein- bis dreifach, gleich oder verschieden durch Alkyl mit 1 bis 4 Kohlenstoffatomen substituiertes Cycloalkyl mit 3 bis 8 Kohlenstoffatomen steht, wobei der Cycloalkylring zusätzlich einen ankondensierten Ring enthalten kann, für gegebenenfalls ein- bis fünffach, gleich oder verschieden durch Halogen, Alkyl mit 1 bis 4 Kohlenstoffatomen, Halogenalkyl mit 1 bis 4 Kohlenstoffatomen und 1 bis 8 gleichen oder verschiedenen Halogenatomen, Nitro, Cyano, Alkoxy mit 1 bis 4 Kohlenstoffatomen oder Alkylmercapto mit 1 bis 4 Kohlenstoffatomen substituiertes Phenylalkyl mit 1 bis 4 Kohlenstoffatomen im Alkylteil, wobei der Alkylteil gegebenenfalls einen weiteren Phenylrest als Substituenten enthält, der gegebenenfalls, wie vorher angegeben, substituiert sein kann, oder für gegebenenfalls ein- bis fünffach, gleich oder verschieden durch Halogen, Nitro, Alkyl mit 1 bis 12 Kohlenstoffatomen, Alkoxy, Alkylmercapto, Carbalkoxy, Alkylsulfonylamino, Alkylsulfonyl, Sulfamoyl, N-Alkylsulfamoyl, N,N-Dialkylsulfamoyl, Dialkylamino, Carbamoyl, N-Alkylcarbamoyl, N,N-Dialkylcarbamoyl mit jeweils 1 bis 4 Kohlenstoffatomen je Alkylrest, Halogenalkyl, Halogenalkoxy, Halogenalkylmercapto mit jeweils 1 bis 4 Kohlenstoffatomen und 1 bis 8 gleichen oder verschiedenen Halogenatomen je aufgezähltem Rest, Phenyl, Phenoxy, Phenylmercapto, Acyloxy mit 1 bis 3 Kohlenstoffatomen, Acyl mit 1 bis 3 Kohlenstoffatomen, Phenylalkyloxy mit 1 bis 3 Kohlenstoffatomen im Alkylteil, Phenylalkylmercapto mit 1 bis 3 Kohlenstoffatomen, Acylamino mit 1 bis 3 Kohlenstoffatomen, Acylalkylamino mit 1 bis 3 Kohlenstoffatomen je Acyl- und Alkylrest und Cycloalkyl mit 4 bis 6 Kohlenstoffatomen und Cyan substituiertes Phenyl oder für Naphthyl steht oder
R und $R^1$ gemeinsam mit dem Stickstoffatom, an dem sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann,
mit der Ausnahme, daß R nicht Wasserstoff ist, wenn A für SO oder $SO_2$ steht.

Besonders bevorzugt sind Verbindungen der Formel (I), in welcher
Hal für Chlor, Brom oder Iod steht,
A für O, S, SO, $SO_2$ oder $NR^1$ steht, wobei
$R^1$ für Wasserstoff, Alkyl mit 1 bis 4 Kohlenstoffatomen, Alkenyl mit 3 oder 4 Kohlenstoffatomen, für gegebenenfalls ein- bis dreifach durch Alkyl mit 1 bis 3 Kohlenstoffatomen substituiertes Phenyl, Benzyl oder 2-Phenyl-2-methylethyl steht und
R für Wasserstoff, Alkyl mit 1 bis 8 Kohlenstoffatomen, Alkenyl mit 3 bis 8 Kohlenstoffatomen, Alkinyl mit 4 bis 8 Kohlenstoffatomen, Cyanalkyl mit 1 bis 3 Kohlenstoffatomen im Alkylteil, Cycloalkyl mit 3 bis 6 Kohlenstoffatomen, oder für gegebenenfalls ein- bis fünffach, gleich oder verschieden durch Alkyl mit 1 bis 12 Kohlenstoffatomen, Alkenyl mit bis zu 3 Kohlenstoffatomen, Fluor, Chlor, Brom, Iod, Trifluormethyl, Nitro, Alkoxy mit 1 bis 4 Kohlenstoffatomen, Alkylmercapto mit 1 bis 4 Kohlenstoffatomen, Dialkylamino mit 1 oder 2 Kohlenstoffatomen je Alkylrest, Carbalkoxy mit 1 oder 2 Kohlenstoffatomen, Trifluormethoxy, Trifluormethylmercapto, Alkylsulfonylamino mit 1 oder 2 Kohlenstoffatomen, Alkylsulfonyl mit 1 oder 2 Kohlenstoffatomen, Phenyl, Phenoxy, Phenylmercapto, Acetoxy, Acetyl, Carbamoyl, Sulfamoyl, N,N-Dialkylsulfamoyl mit 1 oder 2 Kohlenstoffatomen je Alkylrest, Benzyloxy, Benzylmercapto, Formylamino, Formylmethylamino, Acetylamino, Cyclopentyl, Cyclohexyl oder Cyano substituiertes Phenyl, Benzyl oder Phenethyl oder Naphthyl steht oder
R und $R^1$ gemeinsam mit dem Stickstoffatom, an dem sie stehen, für gegebenenfalls ein- oder zweifach durch Alkyl mit 1 oder 2 Kohlenstoffatomen substituiertes Pyrrolidin, Piperidin, Hexamethylenimin, Morpho-

lin, N-Alkylpiperazin ($C_1$-$C_2$), Pyrrol, Pyrazol, Imidazol oder 1,2,4-Triazol stehen, mit der Ausnahme, daß R nicht Wasserstoff ist, wenn A für SO oder $SO_2$ steht.

Ganz besonders hervorzuheben sind Verbindungen der Formel (I), in welcher

Hal für Halogen steht und

A-R für OR steht, worin

R für Alkyl, Alkenyl, Alkinyl steht, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht,

oder

A-R für SR steht, worin

R für Alkyl, Alkenyl oder Alkinyl steht, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkoxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkoxy, Aralkylmercapto, Acylamino, Acylalkylamino, Cycloalkyl und Cyan substituiertes Aryl steht,

oder

A-R für $SO_nR$ steht, worin

n für 1 oder 2 steht und

R für gegebenenfalls ein- bis mehrfach, gleich oder verschieden durch Halogen, Alkyl, Alkenyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkoxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht,

oder

A-R für -NH-R steht, worin

R für Alkenyl, Alkinyl, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Halogenalkyl, Nitro, Dialkylamino, Carbalkoxy, Carbamoyl, N-Alkylcarbamoyl, N,N-Dialkylcarbamoyl, Halogenmethyloxy, Halogenmethylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht,

oder

A-R für

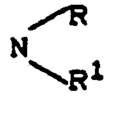

steht, worin

$R^1$ für Alkyl, Alkenyl, Halogenalkyl, Cyanalkyl oder für jeweils gegebenenfalls ein- bis mehrfach, gleich oder verschieden substituiertes Cycloalkyl, Aryl oder Aralkyl steht und

R für Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano

substituiert sein können, für gegebenenfalls ein- bis mehrfach, gleich oder verschieden durch Alkyl substituiertes Cycloalkyl steht, wobei zusätzlich zu der Alkylsubstitution ein Ring an kondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Halogenalkyl, Nitro, Dialkylamino, Carbalkoxy, Carbamoyl, N-Alkylcarbamoyl, N-N-Dialkylcarbamoyl, Halogenmethyloxy, Halogenmethylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht oder

R und $R^1$ zusammen mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann.

Die Kohlenstoffatomanzahl, Substituenten und Substituentenanzahl entspricht den Angaben bei der bevorzugten Definition.

Verwendet man bei dem erfindungsgemäßen Verfahren (a) 2,4-Dichlor-5-nitrothiazol und Anilin als Ausgangsstoffe, so kann der Verlauf der Umsetzung durch das folgende Formelschema wiedergegeben werden:

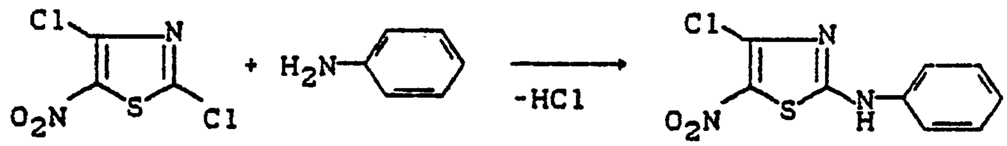


Verwendet man 2,4-Dichlor-5-nitrothiazol und N,N-Dimethylanilin als Ausgangsstoffe, so kann der Verlauf des erfindungsgemäßen Verfahrens (b) durch das folgende Formelschema wiedergegeben werden:

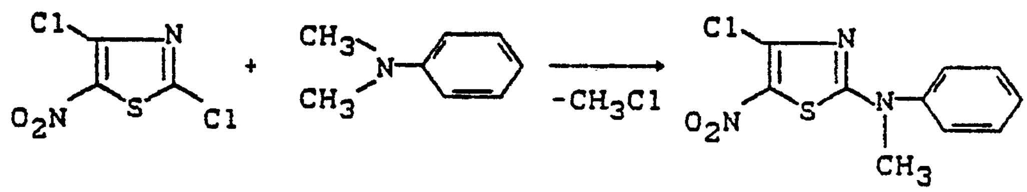


Verwendet man 4-Chlor-2-(4-methylphenylmercapto)-5-nitrothiazol und 2 Mol 3-Chlorperoxybenzoesäure als Ausgangsstoffe, so kann der Verlauf des erfindungsgemäßen Verfahrens (c), Variante α, durch das folgende Formelschema wiedergegeben werden:

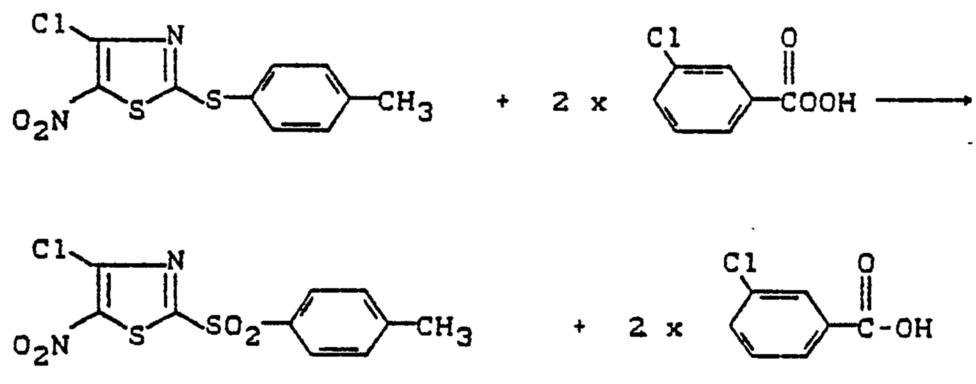

Verwendet man 4-Chlor-2-(4-methylphenylmercapto)-5-nitrothiazol als Ausgangsstoff und 1 Mol 3-Chlorperoxybenzoesäure als Oxidationsmittel, so kann der Verlauf des erfindungsgemäßen Verfahrens (c), Variante β, durch das folgende Formelschema wiedergegeben werden:

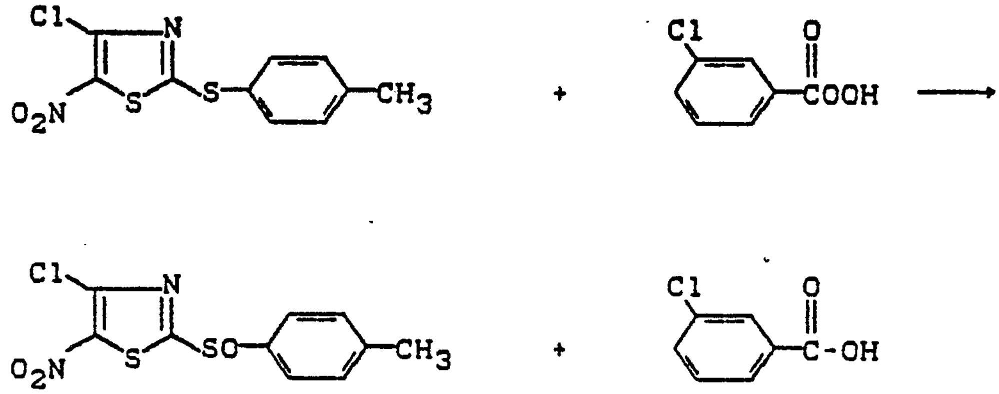

Die bei dem erfindungsgemäßen Verfahren (a) als Ausgangsprodukte benötigten 2,4-Dihalogen-5-nitrothiazole der Formel (II) sind teilweise bekannt, z. B. für Verbindungen der Formel (II), in welcher
Hal = $Hal^1$ = Brom steht: Chem. Abstr. 61: 3087 f
und für
Hal = $Hal^1$ = Chlor steht, vergl. DE-OS 3 518 520.

Die beiden genannten Verbindungen sind z. B. durch Nitrierung der entsprechenden 2,4-Dihalogenthiazole zugänglich.

Verbindungen der Formel (II A)

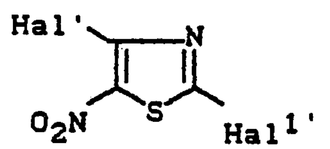

(II A)

in welcher
Hal′ und $Hal^{1}$′ unabhängig voneinander für Chlor, Iod oder Fluor stehen, mit der Maßgabe, daß nicht beide Hals gleichzeitig Chlor sein dürfen, sind neu und ebenfalls Teil der Erfindung. Bei entsprechenden Anwendungskonzentrationen zeigen auch diese Stoffe eine mikrobizide Wirkung.

So sind Verbindungen der Formel (II A), in der
Hal′ = $Hal^1$′ = Iod oder
Hal′ = Chlor und $Hal^1$′ = Iod oder
Hal′ = Iod und $Hal^1$′ = Chlor oder
Hal′ = Chlor und $Hal^1$′ = Fluor
bedeuten, z. B. neu. So werden die Iodverbindungen durch Umsetzung von 2,4-Dichlor-5-nitrothiazol mit Metalliodiden, insbesondere Natriumiodid, in niederen aliphatischen Ketonen, insbesondere Aceton, als Lösungsmittel entsprechend den folgenden Formelschemata gewonnen:

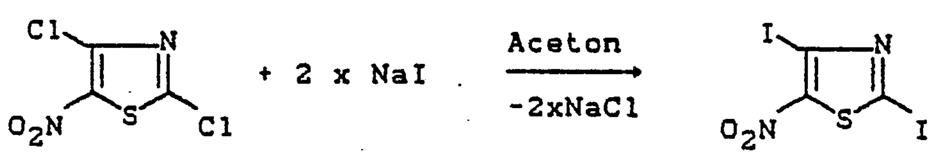


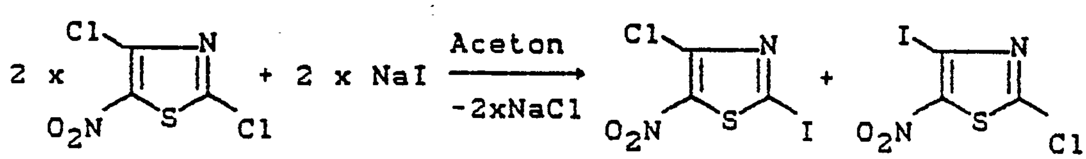


Die beiden isomeren Chlor-iod-nitrothiazole entstehen nebeneinander und können durch fraktionierte Kristallisation und/oder durch chromatographische Methoden voneinander getrennt werden. Die Reaktionstemperatur liegt im allgemeinen zwischen 0 ° C und 130 ° C, vorzugsweise zwischen 10 ° C und 90 ° C.

Verbindungen der Formel (II A), in der mindestens eines der Halogene Fluor ist, z. B. $Hal^1$ = Fluor und $Hal'$ = Chlor, also das 4-Chlor-2-fluor-5-nitrothiazol können hergestellt werden, indem man 2,4-Dichlor-5-nitrothiazol mit Metallfluoriden, insbesondere Natriumfluorid oder Kaliumfluorid, in niederen aliphatischen Nitrilen, insbesondere in Acetonitril oder Propionitril als Lösungsmittel in Gegenwart katalytischer Mengen von Kronenethern, insbesondere von [18] Krone-6 umsetzt. Die Reaktionstemperatur beträgt 0 ° C bis 50 ° C, vorzugsweise 10 ° C bis 30 ° C. Man arbeitet mit 2 - 15 mol Metallfluorid, vorzugsweise 3 - 12 mol Metallfluorid pro Mol 2,4-Dichlor-5-nitrothiazol.

Die bei dem erfindungsgemäßen Verfahren (a) weiterhin als Ausgangsprodukte benötigten Nucleophile der Formel (III) sind grundsätzlich bekannt. Falls spezielle Verbindungen noch nicht beschrieben sind, lassen sie sich nach bekannten Verfahren herstellen.

Als Lösungsmittel kommen bei der Durchführung des erfindungsgemäßen Verfahrens (a) in Frage: Aliphatische Nitrile, insbesondere Acetonitril, offenkettige oder cyclische Dialkylamide von aliphatischen Carbonsäuren wie Dimethylformamid, N,N-Dimethylacetamid, ferner N-Methyl-2-pyrrolidinon, N-Methylcaprolactam, Tetramethylharnstoff, N,N′-Dimethyl-1,3-imidazolin-2-on, Hexamethylphosphorsäuretrisamid, Dimethylsulfoxid, Dimethylsulfon, Tetramethylensulfon (= Sulfolan), Tetrahydrofuran, Dioxan sowie niedere aliphatische Alkohole, insbesondere in Verbindung mit ihrem zugehörigen Metall-, insbesondere Alkalisalz.

Bevorzugt werden cyclische Ether wie Dioxan und vor allem die aprotischen, polaren Lösungsmittel wie Dimethylformamid und N-Methyl-2-pyrrolidinon sowie Acetonitril verwendet.

Die Wahl der Lösungsmittel richtet sich auch nach der Natur des Nucleophils der Formel (III). Wird das Nucleophil oder dessen Metallsalz, vorzugsweise Alkalisalz, als wäßrige Lösung (wie z. B. in Natronlauge oder Methylamin) eingesetzt, kann man auch ein Mischsystem aus Wasser und einem der genannten Lösungsmittel verwenden.

Schwach basische Amine, wie z. B. Pyrrol, Pyrazol, Diphenylamin oder durch negative Substituenten wie z. B. Nitro oder Trifluormethyl substituierte Anilinderivate, lassen sich besonders vorteilhaft mit aprotischen, polaren Lösungsmitteln wie Dimethylformamid oder N-Methyl-2-pyrrolidinon umsetzen, während starke Basen wie z. B. aliphatische Amine, wie z. B. Diethylamin oder Morpholin auch in Dioxan bzw. Acetonitril schnell reagieren.

Die Reaktionstemperatur kann bei dem erfindungsgemäßen Verfahren (a) in weiten Bereichen variiert werden. Im allgemeinen arbeitet man zwischen - 50 ° C und + 150 ° C, vorzugsweise bei - 20 ° C bis 100 ° C.

Im allgemeinen reagieren starke Nucleophile bereits an der unteren Grenze des genannten Temperaturbereiches sehr schnell im gewünschten Sinne, während schwache Nucleophile höherer Temperaturen und längerer Reaktionszeiten bedürfen.

Die Umsetzungen werden in der Regel bei Normaldruck ausgeführt, jedoch kann auch in geschlossenen Gefäßen gearbeitet werden, in denen sich dann in Abhängigkeit von der Art des Lösungsmittels und von der Temperatur ein entsprechend höherer Druck einstellt.

Zur Bindung des bei dem erfindungsgemäßen Verfahren (a) gemäß Formelschema freiwerdenden Halogenwasserstoffs ist im allgemeinen ein säurebindendes Mittel erforderlich. Hierfür sind z. B. verwendbar: Alkalihydroxide, -carbonate oder -hydrogencarbonate.

Anstelle des säurebindenden Mittels können auch direkt Metallsalze, vorzugsweise Alkalisalze des betreffenden Nucleophils der Formel (III) zur Reaktion gebracht werden. Die Salze können entweder als solche eingesetzt werden oder, beispielsweise durch Zugabe von Alkalihydriden, vorzugsweise Natriumhydrid, in situ erzeugt werden.

Sind die einzusetzenden Nucleophile stark basische Amine wie beispielsweise Diethylamin, so kann ein entsprechender Überschuß des Nucleophils als säurebindendes Mittel dienen. Schwächer basische Amine, wie Aniline, die in aprotischen, stark polaren Lösungsmitteln wie Dimethylformamid oder N-Methyl-2-pyrrolidinon umgesetzt werden, liefern ohne Zusatz eines säurebindenden Mittels und ohne Verwendung eines Überschusses des Nucleophiles in guten Ausbeuten die gewünschten Verbindungen der Formel (I).

Sehr schwache Nucleophile der Formel (III), beispielsweise zwei- bis mehrfach durch negative Substituenten substituierte Aniline, insbesondere solche, die in den ortho-Stellungen zur Aminogruppe diese negativen Substituenten haben, werden zweckmäßig in Ethern, insbesondere in cyclischen Ethern wie beispielsweise Tetrahydrofuran oder Dioxan unter Zugabe von Metallhydriden, vorzugsweise Natriumhydrid, zwecks Salzbildung in situ, umgesetzt.

Die Reaktionspartner werden im allgemeinen im stöchiometrisch äquimolaren Verhältnis miteinander zur Reaktion gebracht, im Falle stark basischer Amine, die gleichzeitig als säurebindendes Agens fungieren können, in zweifach molarem Verhältnis.

Da die 2,4-Dihalogen-5-nitrothiazole der Formel (II) in 4-Stellung ein zweites - wenn auch abgestuft deutlich weniger reaktives - Halogenatom tragen, ist es im allgemeinen zweckmäßig, keinen Überschuß an

Nucleophil der Formel (III) einzusetzen. In bestimmten Fällen jedoch kann es vorteilhaft sein, einem Überschuß an 2,4-Dihalogen-5-nitrothiazol der Formel (II) einzusetzen, der im allgemeinen 20 Molprozent bis zu 100 Molprozent betragen kann. Bei schwachen Nucleophilen der Formel (III), die mit dem wesentlich reaktiveren Halogen in 2-Stellung des Thiazolrings nur relativ langsam reagieren, kann dagegen ein Überschuß des Nucleophils der Formel (III) eingesetzt werden, der bis zu 100 Molprozent betragen kann.

Bei der Durchführung des erfindungsgemäßen Verfahrens (a) wird bevorzugt das 2,4-Dihalogen-5-nitrothiazol der Formel (II) in einem der genannten Lösungsmittel vorgelegt und dann gegebenenfalls das säurebindende Mittel zugesetzt. Anschließend fügt man bei Raumtemperatur oder darunter, besonders bevorzugt bis - 15 °C, das Nucleophil der Formel (III) langsam in kleinen Portionen, z. B. durch Zutropfen einer Lösung bzw. in einem Verdünnungsmittel zu. Durch diese Maßnahme des langsamen Zugebens werden lokale Überschüsse an Nucleophil der Formel (III) während der Umsetzung mit dem 2,4-Dihalogen-5-nitrothiazol der Formel (II) weitestgehend vermieden und damit die Bildung hier unerwünschter Disubstitutionsprodukte praktisch ausgeschlossen.

Soll die gewünschte End-Reaktionstemperatur oberhalb Raumtemperatur liegen, wird anschließend erhitzt.

Die Isolierung der Reaktionsprodukte erfolgt nach üblichen Methoden. Im einfachsten Fall wird das Reaktionsgemisch gegebenenfalls nach teilweisem oder völligem Abdestillieren des Lösungsmittels im Vakuum unterhalb der Reaktionstemperatur in überschüssigem Eiswasser, etwa der 5- bis 10-fachen Volumenmenge des eingesetzten Lösungsmittels, verrührt, abfiltriert, mit Wasser gewaschen und getrocknet. Wenn mit einem Überschuß 2,4-Dihalogen-5-nitrothiazol gearbeitet wurde, kann dies aus dem gut getrockneten Rohprodukt durch Verrühren bei Raumtemperatur mit einem Lösungsmittel, das praktisch nur das 2,4-Dihalogen-5-nitrothiazol der Formel (II) löst, herausgelöst und gegebenenfalls für Folgeansätze wiederverwendet werden. Im Falle des 2,4-Dichlor-5-nitrothiazols ist z. B. Petrolether besonders gut geeignet. Falls das Reaktionsprodukt, das noch überschüssiges 2,4-Dihalogen-5-nitrothiazol der Formel (II) enthält, selbst in Petrolether bei Raumtemperatur gut löslich ist, wird das 2,4-Dihalogen-5-nitrothiazol der Formel (II) z. B. im Vakuum bei etwa 0,1 mbar fraktioniert von dem meist deutlich schwerer flüchtigen Reaktionsprodukt absublimiert bzw. abdestilliert. Im Falle des 2,4-Dichlor-5-nitrothiazols ist dies z. B. bei einer Temperatur von etwa 70 °C und 0,1 mbar möglich. Eine weitere Reinigung der Reaktionsprodukte kann z. B. durch Umkristallisieren oder auf chromatographischem Wege erfolgen.

Falls ein Reaktionsprodukt beim Verrühren in überschüssigem Eiswasser als Öl anfällt, wird es durch mehrmaliges Ausschütteln mit einem der gebräuchlichen organischen, nicht mit Wasser mischbaren Lösungsmitteln wie z. B. Methylenchlorid isoliert, indem man die organische Phase abtrennt, gegebenenfalls mit Wasser wäscht, z. B. im Falle von Dimethylformamid oder N-Methyl-2-pyrrolidinon als Reaktionsmedium, trocknet und im Vakuum einengt.

Die bei dem erfindungsgemäßen Verfahren (b) als Ausgangsprodukte benötigten N,N-Dimethylarylamine der Formel (IV) sind grundsätzlich bekannt. Falls spezielle Verbindungen noch nicht beschrieben sind, lassen sie sich nach bekannten Verfahren herstellen. In Formel (IV) hat Aryl die gleiche Bedeutung wie Aryl in der Definition von R in der Formel (I).

Als Lösungsmittel kommen bei der Durchführung des erfindungsgemäßen Verfahrens (b) in Frage: Aliphatische oder aromatische Nitrile, z. B. Acetonitril, Propionitril, Benzonitril, insbesondere Acetonitril, Ether, insbesondere cyclische Ether wie Dioxan, Tetrahydrofuran, insbesondere Dioxan.

Die Reaktionstemperatur kann bei dem erfindungsgemäßen Verfahren (b) in weiten Bereichen variiert werden. Im allgemeinen arbeitet man zwischen 50 °C und 200 °C, vorzugsweise bei 60 °C bis 150 °C.

Die Umsetzungen werden in der Regel bei Normaldruck ausgeführt, jedoch kann auch in geschlossenen Gefäßen gearbeitet werden, in denen sich dann in Abhängigkeit von der Art des Lösungsmittels und von der Temperatur ein entsprechend höherer Druck einstellt.

Die Reaktionspartner werden vorzugsweise im stöchiometrisch äquimolaren Verhältnis miteinander zur Reaktion gebracht.

Bei der Durchführung des erfindungsgemäßen Verfahrens (b) wird das 2,4-Dihalogen-5-nitrothiazol der Formel (II) vorzugsweise in einem der genannten Lösungsmittel gelöst, vorzugsweise die etwa äquimolare Menge des N,N-Dimethylarylamins hinzugefügt und so lange, vorzugsweise unter Rückfluß, erhitzt, bis die Umsetzung praktisch beendet ist. Anschließend destilliert man das Lösungsmittel, vorzugsweise im Vakuum, ab und reinigt, falls erforderlich, zum Beispiel durch Umkristallisation.

Die bei dem erfindungsgemäßen Verfahren (c) als Ausgangsstoffe benötigten, erfindungsgemäßen 4-Halogen-5-nitrothiazolylsulfide sind durch die Formel (V) definiert. In dieser Formel stehen Hal und R vorzugsweise für diejenigen Reste, die bereits im Zusammenhang mit der Beschreibung der erfindungsgemäßen Stoffe der Formel (I) genannt wurden.

Als Oxidationsmittel können bei dem erfindungsgemäßen Verfahren (c) alle üblichen für derartige

Zwecke verwendbaren Reagenzien eingesetzt werden. Bevorzugt sind aliphatische oder aromatische Percarbonsäuren wie beispielsweise Peressigsäure, Perpropionsäure, 3-Chlorperbenzoesäure. Besonders bevorzugt ist 3-Chlorperbenzoesäure.

Als Lösungsmittel können bei dem erfindungsgemäßen Verfahren (c) alle üblichen inerten, organischen Solventien eingesetzt werden. Vorzugsweise in Betracht kommen Dichlormethan, Trichlormethan und Tetrachlormethan.

Die Reaktionstemperaturen können bei dem erfindungsgemäßen Verfahren (c) innerhalb eines bestimmten Bereiches variiert werden. Im allgemeinen arbeitet man bei Temperaturen zwischen 0 °C und 100 °C, vorzugsweise zwischen 20 °C und 80 °C.

Bei der Durchführung des erfindungsgemäßen Verfahrens (c), Variante α, löst man im allgemeinen das 4-Halogen-5-nitrothiazolyl-sulfid der Formel (V) in einem der genannten Lösungsmittel und versetzt mit mindestens 2 Mol eines Oxidationsmittels, vorzugsweise einer der genannten Percarbonsäuren, vorzugsweise bis zu 5 Mol Oxidationsmittel pro Mol der Verbindung der Formel (V) und rührt z. B. unter Dünnschichtchromatographie-Verfolgung so lange, bis die gewünschte Umsetzung beendet ist. Zur Beschleunigung der Reaktion wird hierbei vorzugsweise unter Rückflußbedingungen gearbeitet.

Bei der Durchführung des erfindungsgemäßen Verfahrens (c), Variante β, löst man das 4-Halogen-5-nitrothiazolyl-sulfid der Formel (V) in einem der genannten Lösungsmittel und versetzt zur Vermeidung von hierbei unerwünschter Sulfonbildung vorzugsweise im unteren Bereich der genannten Reaktionstemperatur, z. B. bei Raumtemperatur, mit kleinen Portionen Oxidationsmittel, vor zugsweise Percarbonsäure unter Dünnschichtchromatographie-Verfolgung der Umsetzung. Die Gesamtmenge Oxidationsmittel, z. B. der Percarbonsäure, liegt vorzugsweise im Bereich zwischen 1 - 1,5 Mol pro Mol Verbindung der Formel (V). Da die Umsetzung bei niedrigen Temperaturen, z. B. bei Raumtemperatur, naturgemäß relativ langsam abläuft, kann man mit Hilfe der Dünnschicht-Chromatographie auch bei Anwendung von mehr als 1 Mol Percarbonsäure pro Mol Verbindung der Formel (V) die Umsetzung abstoppen, bevor nennenswerte Mengen unerwünschten Sulfons gebildet werden.

Die Reinisolierung der Sulfone bzw. der Sulfoxide erfolgt in üblicher Weise. Man zieht das Lösungsmittel im Vakuum ab, verrührt zur Entfernung z. B. der (Per)-Carbonsäuren bei Raumtemperatur mit überschüssiger wäßriger Natriumhydrogencarbonat-Lösung, filtriert, wäscht mit Wasser, trocknet und kristallisiert gegebenenfalls beispielsweise aus Kohlenwasserstoffen, wie Cyclohexan, um.

Die erfindungsgemäßen Wirkstoffe der Formel (I) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, vor allem als Fungizide, geeignet und außerdem auch für den Gebrauch zum Schutz technischer Materialien gegen Mikroben, wie z.B. Pilze, Bakterien und Schleimorganismen.

Fungizide Mittel im Pflanzenschutz werden eingesetzt zur Bekämpfung von Plasmodiophoromycetes, Oomycetes, Chytri diomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;

Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Obst-, Gemüse-und Getreidekrankheiten, wie z. B. gegen den Erreger des Apfelschorfes (Venturia inaequalis), gegen den Erreger der Braunfäule der Tomaten (Phytophthora infestans), gegen Erreger von Rebenkrankheiten (Plasmopara viticola) oder gegen Erreger von Getreidekrankheiten, wie z. B. Leptosphaeria nodorum, Fasarium nivale und Pyrenophora teres, einsetzen. Auch sei die Wirkung gegen die Erreger der Reisfleckenkrankheit (Pyricularia oryzae) und die breite in-vitro-Wirkung erwähnt. Bei entsprechenden Anwendungskonzentrationen haben viele der erfindungsgemäßen Verbindungen auch eine bakterizide Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Weiterhin sind die erfindungsgemäßen Wirkstoffe der Formel (I) auch zum Schutz technischer Materialien aufgrund ihres breiten Wirkungsspektrums geeignet.

Da technische Materialien von sehr vielen und sehr verschiedenen Mikrobenarten befallen und geschädigt werden können, ist ein breites Wirkungsspektrum, das auch vielfältige Verwendbarkeit ermöglicht, eine unabdingbare Eigenschaft fortschrittlicher Materialschutzstoffe.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vormikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder

Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate, wie 2-Phenylphenol, (2,2′-Dihydroxy-5,5′-dichlor)-diphenylmethan und 3-Methyl-4-chlor-phenol, Organo-Zinnverbindungen.

Herstellungsbeispiele

Beispiel 1

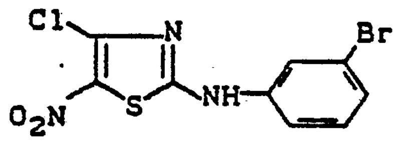

(Verbindung Nr. 51)

Zu einer Lösung von 12,0 g (0,06 mol) 2,4-Dichlor-5-nitrothiazol in 100 ml Dimethylformamid tropft man bei 0 °C bis 5 °C im Verlauf von etwa 2 Stunden eine Lösung von 8,6 g (0,05 mol) 3-Bromanilin in 100 ml Dimethylformamid zu und rührt noch 4 Stunden bei derselben Temperatur nach. Dann wird in 1000 ml Eiswasser eingerührt, der Niederschlag abfiltriert, mit Wasser gewaschen und getrocknet. Zur Entfernung von überschüssigem 2,4-Dichlor-5-nitrothiazol wird bei Raumtemperatur in 80 ml Petrolether verrührt, abfiltriert, mit Petrolether gewaschen und getrocknet. Man erhält so 16,0 g (95,7 % der Theorie) 2-(3-Bromphenylamino)-4-chlor-5-nitrothiazol vom Schmelzpunkt 191 °C bis 192 °C (Zers.)

Beispiel 2

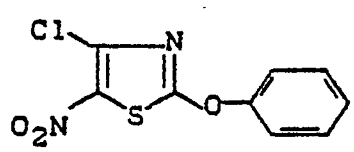

(Verbindung Nr. 52)

Zu einer Lösung von 72,0 g (0,36 mol) 2,4-Dichlor-5-nitrothiazol in 300 ml Acetonitril werden 42 g (0,036 mol) wasserfreies Kaliumcarbonat gegeben. Anschließend tropft man bei 15 °C bis 20 °C im Verlauf von etwa 1,5 Stunden eine Lösung von 28,2 g (0,3 mol) Phenol in 300 ml Acetonitril zu und rührt noch 0,5

Stunden bei derselben Temperatur nach. Anschließend wird 15 Minuten auf Rückflußtemperatur erhitzt, auf Raumtemperatur abgekühlt, in 3 Liter Eiswasser eingerührt, der Niederschlag abfiltriert, mit Wasser gewaschen und getrocknet. Zur Entfernung von überschüssigem 2,4-Dichlor-5-nitrothiazol wird bei etwa 20 °C in 500 ml Petrolether verrührt, abfiltriert, mit Petrolether gewaschen und getrocknet. Man erhält so 75,4 g (98,0 % der Theorie) 4-Chlor-5-nitro-2-phenoxythiazol. Schmelzpunkt 96 °C (aus Petrolether).

Beispiel 3

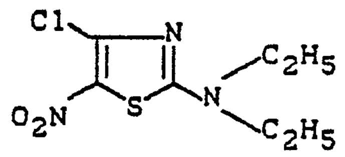

(Verbindung Nr. 1)

Zu einer Lösung von 199 g (1 mol) 2,4-Dichlor-5-nitrothiazol in 1 l Dioxan tropft man bei 20 °C bis 30 °C im Verlauf von etwa 2 Stunden 146 g (2 mol) Diethylamin zu. Nach Stehen über Nacht bei Raumtemperatur wird in 8 l Eiswasser eingerührt, der Niederschlag abfiltriert, mit Wasser gewaschen und getrocknet. Nach erhält 216 g (91,7 % der Theorie) 2-Diethylamino-4-chlor-5-nitrothiazol vom Schmelzpunkt 85 °C (aus Cyclohexan).

Beispiel 4

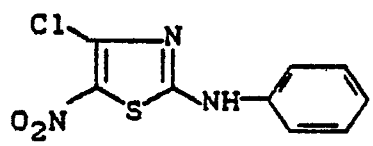

(Verbindung Nr. 5)

Eine Lösung von 360 g (1,8 mol) 2,4-Dichlor-5-nitrothiazol in 1,5 l Acetonitril wird mit 252 g (3,0 mol) Natriumhydrogencarbonat versetzt. Anschließend tropft man bei - 10 °C bis - 15 °C im Verlauf von etwa 4,5 Stunden eine Mischung aus 139,5 g (1,5 mol) Anilin und 1.500 ml Acetonitril zu und rührt weitere 4 Stunden bei derselben Temperatur nach. Dann wird in 15 l Eiswasser eingerührt, der Niederschlag abfiltriert, mit Wasser gewaschen und getrocknet. Zur Entfernung von überschüssigem 2,4-Dichlor-5-nitrothiazol wird bei Raumtemperatur in 2,4 l Petrolether verrührt, abfiltriert, mit Petrolether gewaschen und getrocknet. Man erhält 361,7 g (94,4 % der Theorie) 2-Anilino-4-chlor-5-nitrothiazol vom Schmelzpunkt 186 °C bis 187 °C (Zers.).

Beispiel 5

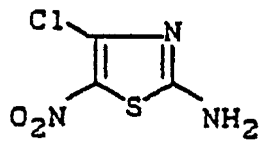

(Verbindung Nr. 3)

Eine Lösung von 19,9 g (0,1 mol) 2,4-Dichlor-5-nitrothiazol in 200 ml Acetonitril wird unter Rühren mit 10,1 g (0,105 mol) Ammoniumcarbonat versetzt und zwei Tage bei Raumtemperatur weitergerührt. Anschließend wird in 1 l Eiswasser eingerührt, der Niederschlag abfiltriert, mit Wasser gewaschen und getrocknet. Zur Entfernung von nicht umgesetztem 2,4-Dichlor-5-nitrothiazol wird bei Raumtemperatur in etwa 100 ml Petrolether verrührt, abfiltriert, mit Petrolether gewaschen und getrocknet. Man erhält 12,2 g (68,0 % der Theorie) 2-Amino-4-chlor-5-nitrothiazol vom Schmelzpunkt 180 °C (Zers.).

Beispiel 6

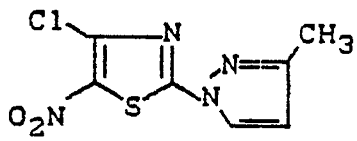

(Verbindung Nr. 11)

Eine Lösung von 5,0 g (0,025 mol) 2,4-Dichlor-5-nitrothiazol in 50 ml N-Methyl-2-pyrrolidinon wird bei Raumtemperatur mit 4,1 g (0,05 mol) 3-Methylpyrazol versetzt. Nach viertägigem Stehen bei Raumtemperatur wird in 500 ml Eiswasser eingerührt, der Niederschlag abfiltriert, mit Wasser gewaschen und getrocknet. Man erhält 5,8 g (94,9 % der Theorie) 4-Chlor-2-(3-methyl-1-pyrazolyl)-5-nitrothiazol vom Schmelzpunkt 153 °C.

Beispiel 7

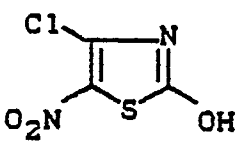 bzw. 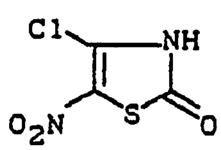

(Verbindung Nr. 17)

Zu einer Lösung von 19,9 g (0,1 mol) 2,4-Dichlor-5-nitrothiazol in 200 ml Dioxan tropft man langsam bei 20 °C bis 25 °C 200 ml (0,2 mol) einer wäßrigen 1n-Natriumhydroxid-Lösung. Nach dem Weiterrühren über Nacht bei Raumtemperatur wird bei 0 °C bis 5 °C mit konzentrierter Salzsäure angesäuert. Durch Einengen der entstandenen Suspension im Vakuum bei Raumtemperatur auf etwa ein Viertel ihres Volumens erhält man insgesamt 13,3 g (73,7 % der Theorie) 4-Chlor-2-hydroxy-5-nitrothiazol bzw. die oben formulierte desmotrope Keto-Form vom Schmelzpunkt 136 °C (Zers.).

Beispiel 8

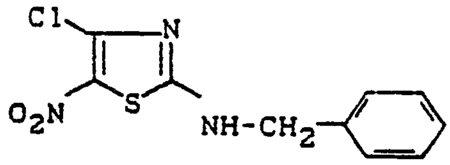

(Verbindung Nr. 18)

Zu einer Lösung von 12,0 g (0,06 mol) 2,4-Dichlor-5-nitrothiazol in 50 ml Acetonitril werden 7,0 g (0,051 mol) wasserfreies Kaliumcarbonat gegeben. Anschließend tropft man bei 15° C bis 20° C im Verlauf von etwa einer halben Stunde eine Mischung aus 5,35 g (0,05 mol) Benzylamin und 50 ml Acetonitril zu und rührt eine weitere Stunde bei Raumtemperatur nach. Anschließend wird 15 Minuten auf Rückflußtemperatur erhitzt, auf Raumtemperatur abgekühlt, in etwa 500 ml Eiswasser eingerührt, der Niederschlag abfiltriert, mit Wasser gewaschen und getrocknet. Zur Entfernung von überschüssigem 2,4-Dichlor-5-nitrothiazol wird bei Raumtemperatur in 80 ml Petrolether verrührt, abfiltriert, mit Petrolether gewaschen und getrocknet. Man erhält 12,9 g (95,7 % der Theorie) 2-Benzylamino-4-chlor-5-nitrothiazol vom Schmelzpunkt 195° C bis 196° C (Zers.).

Beispiel 9

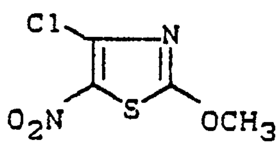

(Verbindung Nr. 45)

Zu einer Lösung von 19,9 g (0,1 mol) 2,4-Dichlor-5-nitrothiazol in 100 ml Methanol tropft man bei 0° C bis 5° C in etwa 1,5 Stunden eine Mischung aus 28,8 g (0,16 mol) 30 %ige Natriummethanolatlösung in Methanol und 40 ml Methanol zu und rührt noch etwa eine Stunde bei Raumtemperatur nach. Nach dem Abziehen des Lösungsmittels bei Raumtemperatur im Vakuum wird der Rückstand mit einer Mischung aus 180 ml 1n-Salzsäure und 90 ml Wasser verrührt, der Niederschlag abfiltriert, mit Wasser gewaschen und getrocknet. Ausbeute: 9,9 g (50,9 % der Theorie)4-Chlor-2-methoxy-5-nitrothiazol vom Schmelzpunkt 52° C (aus Petrolether).

Beispiel 10

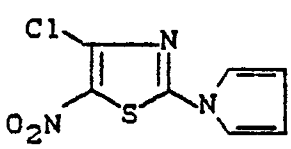

(Verbindung Nr. 67)

Eine Lösung von 10,0 g (0,05 mol)2,4-Dichlor-5-nitrothiazol in 100 ml N-Methyl-2-pyrrolidinon wird bei Raumtemperatur mit 6,7 g (0,1 mol) Pyrrol versetzt. Anschließend wird 5 Stunden auf 70° C erhitzt, dann auf Raumtemperatur abgekühlt und in 1 l Eiswasser eingerührt. Der entstandene Niederschlag wird abfiltriert, mit Wasser gewaschen und getrocknet. Man erhält 8,25 g (72,0 % der Theorie) 4-Chlor-5-nitro-2-(1-pyrrolyl)thiazol vom Schmelzpunkt 138° C bis 139° C (gelbe Nadeln aus Toluol).

Beispiel 11

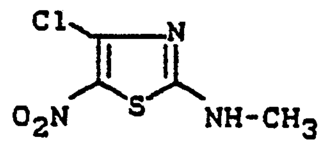

(Verbindung Nr. 18)

Zu einer Lösung von 99,5 g (0,5 mol) 2,4-Dichlor-5-nitrothiazol in 1.000 ml Acetonitril tropft man langsam bei - 10 °C bis 0 °C 48,6 g (0,5 mol) 31,9 %ige wäßrige Methylamin-Lösung, wobei ein Niederschlag ausfällt. Nach dem Weiterrühren über Nacht bei Raumtemperatur wird in 5 l Eiswasser eingerührt, der Niederschlag abfiltriert, mit Wasser gewaschen und getrocknet. Nach Verrühren mit etwa 500 ml Petrolether bei Raumtemperatur wird abfiltriert, mit Petrolether, gewaschen und getrocknet. Man erhält 46,9 g (48,5 % der Theorie, bezogen auf 2,4-Dichlor-5-nitrothiazol bzw. 97 % der Theorie, bezogen auf Methylamin) 4-Chlor-2-methylamino-5-nitrothiazol vom Schmelzpunkt 212 °C (Zers.). Die Verbindung kann aus Methanol umkristallisiert werden und ist bei 140 °C / 0,1 mbar sublimierbar.

Beispiel 12

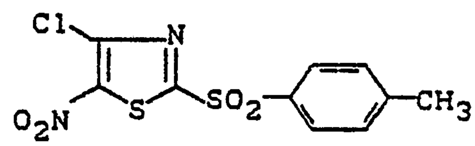

(Verbindung Nr. 66)

Zu einer Lösung von 19,9 g (0,1 mol) 2,4-Dichlor-5-nitrothiazol in 500 ml Dimethylformamid tropft man langsam bei 0 °C bis 5 °C eine durch Erwärmen hergestellte Lösung aus 8,9 g (0,05 mol)

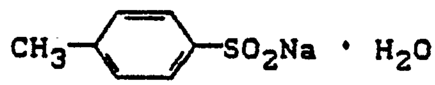

in 50 ml Dimethylformamid, wobei ein Niederschlag ausfällt. Nach sechsstündigem Nachrühren bei 0 °C bis 5 °C wird in 5 l Eiswasser eingerührt, abfiltriert, mit Wasser gewaschen und getrocknet. Man erhält 9,5 g (59,7 % der Theorie) 4-Chlor-2-(4-methylphenylsulfonyl)-5-nitrothiazol. Die Verbindung kann aus Cyclohexan umkristallisiert werden und ist bei 140 °C / 0,1 mbar sublimierbar. Schmelzpunkt 153 °C bis 153,5 °C.

Beispiel 13

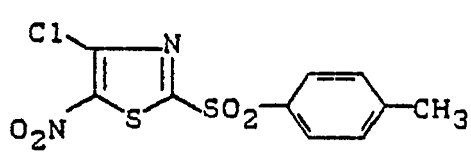

(Verbindung Nr. 66)

Eine Lösung von 5,73 g (0,02 mol) 4-Chlor-2-(4-methylphenylmercapto)-5-nitrothiazol (erfindungsgemäße Verbindung Nr. 57) in 200 ml Dichlormethan wird mit 15,65 g (0,09 mol) 3-Chlorperoxybenzoesäure versetzt und 8 Stunden unter Rückfluß gerührt. Nach dem Erkalten wird zunächst von ausgefallener 3-Chlorbenzoesäure abfiltriert. Das Filtrat wird bei Raumtemperatur im Vakuum zur Trockne eingeengt und der verbleibende Rückstand mit überschüssiger wäßriger Natriumhydrogencarbonat-Lösung intensiv verrührt, abfiltriert, mit Wasser gewaschen und getrocknet. Man erhält 6,2 g (97,3 % der Theorie) 4-Chlor-2-(4-methylphenylsulfonyl)-5-nitrothiazol, das in allen Eigenschaften mit der in Beispiel 12 gewonnenen Verbindung identisch ist.

Beispiel 14

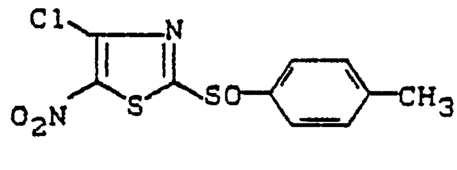

(Verbindung Nr. 73)

Eine Lösung von 5,2 g (0,018 mol) 4-Chlor-2-(4-methylphenylmercapto)-5-nitrothiazol (erfindungsgemäße Verbindung Nr. 57) in 200 ml Dichlormethan wird bei Raumtemperatur im Verlauf von etwa 50 Stunden portionsweise (ca. 8 Portionen) mit insgesamt 4,35 g (0,025 mol) 3-Chlorperoxybenzoesäure versetzt. Danach wird im Vakuum bei Raumtemperatur zur Trockne eingeengt, der verbleibende Rückstand mit überschüssiger wäßriger Natriumhydrogencarbonat-Lösung intensiv verrührt, abfiltriert, mit Wasser gewaschen und getrocknet. Man erhält 4-Chlor-2-(4-methylphenylsulfinyl)-5-nitrothiazol vom Schmelzpunkt 112° C (aus Cyclohexan).

Beispiel 15

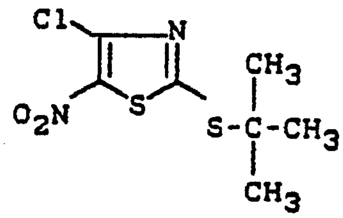

(Verbindung Nr. 107)

Die Herstellung der Verbindung Nr. 107 erfolgt analog Beispiel 2 aus 2,4-Dichlor-5-nitrothiazol und t-Butylmercaptan. Das nach dem Einrühren in überschüssiges Eiswasser entstehende Öl wird in Dichlormethan aufgenommen, die Dichlormethanphase abgetrennt, mit wasserfreiem Natriumsulfat getrocknet und im Vakuum eingeengt. Überschüssiges 2,4-Dichlor-5-nitrothiazol wird durch eine Hochvakuumdestillation in einer Kurzwegapparatur bis zu einer Heizbadtemperatur von 70° C bei 0,1 mbar entfernt, anschließend bei einer Heizbadtemperatur von etwa 80° C / 0,1 mbar reines 2-tert.-Butylmercapto-4-chlor-5-nitrothiazol destilliert.

Beispiel 16

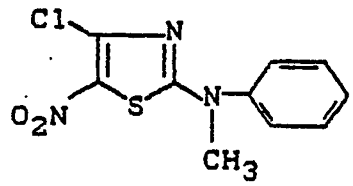

(Verbindung Nr. 13)

Eine Lösung von 19,9 g (0,1 mol) 2,4-Dichlor-5-nitrothiazol in 250 ml Acetonitril wird mit 12,7 g (0,105 mol) N,N-Dimethylanilin versetzt und die Reaktionsmischung 75 Stunden unter Rückfluß gerührt. Danach wird das Lösungsmittel im Vakuum abgezogen, der feste Rückstand in Wasser verrührt, abfiltriert, mit Wasser gewaschen und getrocknet. Man erhält 25,8 g (95,7 % der Theorie) 4-Chlor-2-(N-methyl-N-phenylamino)-5-nitrothiazol. Die Verbindung ist aus Cyclohexan umkristallisierbar und bei etwa 110° C bis 120° C / 0,1 mbar sublimierbar. Schmelzpunkt 136° C bis 137° C.

Eine Herstellung von Verbindung Nr. 13 analog Beispiel 2 ist ebenfalls möglich.

Herstellung bisher nicht literaturbekannter Ausgangsprodukte

Beispiel A1

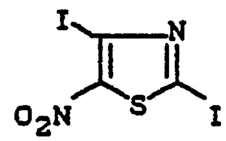

19,9 g (0,1 mol) 2,4-Dichlor-5-nitrothiazol werden in 500 ml Aceton gelöst und nach Zugabe von 150 g (1 mol) Natriumiodid wird etwa 60 Stunden unter Rückfluß gerührt. Danach liegt der Anteil an 2,4-Diiod-5-nitrothiazol im Reaktionsgemisch nach der gaschromatographischen Analyse bei über 95 %. Daraufhin wird das Lösungsmittel im Vakuum abgezogen, der Rückstand in Wasser verrührt, abfiltriert, mit Wasser gewaschen und getrocknet. Man erhält 33,9 g (89 % der Theorie) 2,4-Diiod-5-nitrothiazol vom Schmelzpunkt 135° C nach dem Umkristallisieren aus Cyclohexan.

Beispiel A2

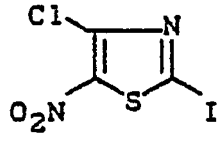

und

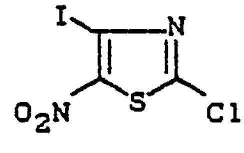

39,8 g (0,2 mol) 2,4-Dichlor-5-nitrothiazol werden in 500 ml Aceton gelöst, und nach Zugabe von 90 g (0,6 mol) Natriumiodid wird etwa 6 Tage bei Raumtemperatur gerührt, wobei der Fortgang der Umsetzung gaschromatographisch verfolgt wird. Dann wird das Lösungsmittel im Vakuum abgezogen, der Rückstand in Wasser verrührt, abfiltriert, mit Wasser gewaschen und getrocknet. Man erhält ein Substanzgemisch, das nach der gaschromatographischen Analyse 71,5 % 2-Iod-4-chlor-5-nitrothiazol und 25,8 % 2-Chlor-4-iod-5-nitrothiazol enthält. HPLC-(= High pressure liquid chromatography) chromatographische Trennung lieferte die Reinsubstanzen mit den folgenden Schmelzpunkten:

2-Iod-4-chlor-5-nitrothiazol: Schmelzpunkt 80° C - 81° C,

2-Chlor-4-iod-5-nitrothiazol: Schmelzpunkt 108° C -109,5° C.

Beispiel A3

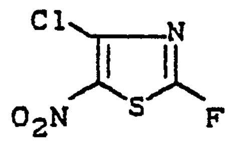

19,9 g (0,1 mol) 2,4-Dichlor-5-nitrothiazol werden in 60 ml Acetonitril gelöst, und nach Zugabe von 24 g (0,4 mol) geglühtem Kaliumfluorid und 0,5 g [18]Krone-6 wird etwa eine Woche bei Raumtemperatur gerührt, wobei der Fortgang der Umsetzung gaschromatographisch verfolgt wird. Der Rohansatz wird bei 0,1 bis 0,2 mbar und bei einer Temperatur von 20 bis 25 °C destilliert, wobei die flüchtigen Bestandteile in einer mit Methanol/Trockeneis gekühlten Vorlage aufgefangen werden. Das Acetonitril wird anschließend bei gewöhnlichem Druck abdestilliert, wobei ein Gemisch aus etwa gleichen Teilen 4-Chlor-2-fluor-5-nitrothiazol und 2,4-Dichlor-5-nitrothiazol zurückbleibt. Hieraus läßt sich das 4-Chlor-2-fluor-5-nitrothiazol zum Beispiel durch fraktionierte Destillation abtrennen.

$^{19}F$-NMR ($CDCl_3$) ($CF_3$-COOH als externer Standard): $\delta$ = -17,5 ppm.
MS: 182 (37%) = $M^+$ = $C_3ClFN_2O_2S$
124 (34%)
91 (60%)
63 (100%)

Als Nebenkomponente in einem relativen Anteil zwischen 1 und 5%, bezogen auf 4-Chlor-2-fluor-5-nitrothiazol kann durch Gaschromatographie das isomere 2-Chlor-4-fluor-5-nitrothiazol identifiziert werden.

$^{19}F$-NMR ($CDCl_3$) ($CF_3$-COOH als externer Standard): $\delta$ = - 22,7 ppm.
MS: 182 (83%) = $M^+$ = $C_3ClFN_2O_2S$
136 (32%)
91 (43%)
75 (100%)

In entsprechender Weise und gemäß den allgemeinen Angaben zur Herstellung erhält man die folgenden 4-Halogen-5-nitrothiazol-Derivate der allgemeinen Formel (I).

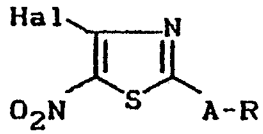

(I)

| Verbindungs-Nummer | Hal | - A - | - R | Schmp. (°C) (umkrist. aus) | Herstellung |
|---|---|---|---|---|---|
| 1 | Cl | 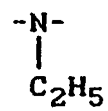 | $-C_2H_5$ | 85 (Cyclohexan) | s. Beispiel 3 |
| 2 | Cl | -A-R = | 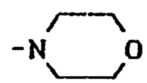 | 135 (Ethanol) | analog Beispiel 3 |
| 3 | Cl | -NH- | H | 180 (Z.) | s. Beispiel 5 |
| 4 | Cl | -A-R = | 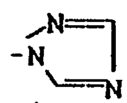 | 122 (Cyclohexan) | analog Beispiel 6 |
| 5 | Cl | -NH- | 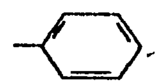 | 190-1 (Z.) | analog Beispiel 1 und s. Beispiel 4 |

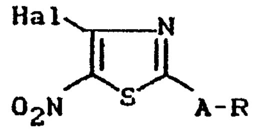

(I)

| Verbin-dungs-Nummer | Hal | - A - | - R | Schmp. (°C) (umkrist. aus) | Herstellung |
|---|---|---|---|---|---|
| 6 | Cl | -A-R = 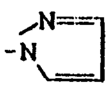 | | 147 (Cyclohexan) | analog Beispiel 6 |
| 7 | Cl | -A-R = 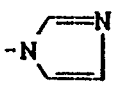 | | 150 (Z.) | analog Beispiel 6 |
| 8 | Cl | -NH- | 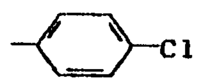 | 207 (Z.) | analog Beispiel 4 |
| 9 | Cl | - O - | 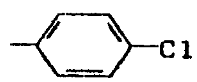 | 94 (Methanol) | analog Beispiel 2 |
| 10 | Cl | -NH- | 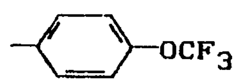 | 121 (Z.) | analog Beispiel 1 |
| 11 | Cl | -A-R = 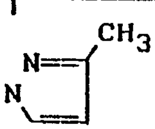 | | 153 | s. Beispiel 6 |

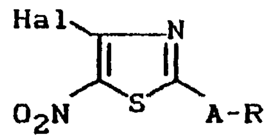

(I)

| Verbin-dungs-Nummer | Hal | - A - | - R | Schmp. (°C) (umkrist. aus) | Herstellung |
|---|---|---|---|---|---|
| 12 | Cl | -A-R = 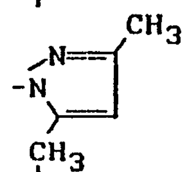 | | 205 | analog Beispiel 6 |
| 13 | Cl | -N-<br>$CH_3$ | 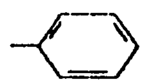 | 136 - 7 (Cyclohexan) | analog Beispiel 2 und s. Beispiel 16 |
| 14 | Cl | -N-<br>$C_2H_5$ | 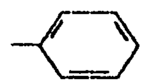 | 93 - 4 (Cyclohexan) | analog Beispiel 2 |
| 15 | Cl | -N-<br>$C_2H_5$ | 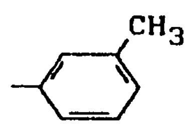 | 81 | analog Beispiel 2 |
| 16 | Cl | -N-<br>$CH_3$ | 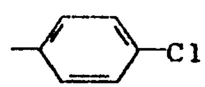 | 118 - 20 | analog Beispiel 1 |

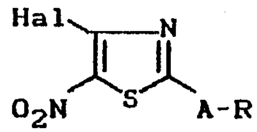

(I)

| Verbin-dungs-Nummer | Hal | - A - | - R | Schmp. (°C) (umkrist. aus) | Herstellung |
|---|---|---|---|---|---|
| 17 | Cl | - O - | H | 136 (Z.) | s. Beispiel 7 liegt als 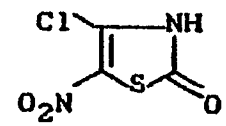 vor |
| 18 | Cl | -NH- | $-CH_2$- 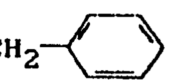 | 195 - 6 (Z.) | s. Beispiel 8 |
| 19 | Cl | -NH- | 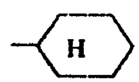 | 130 - 1 (Cyclohexan) | analog Beispiel 2 |
| 20 | Cl | $-N(CH_3)-$ | $-CH_2$- 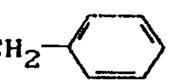 | 112 - 3 (Cyclohexan) | analog Beispiel 2 |
| 21 | Cl | -NH- | $-C(CH_3)_3$ | 175 - 6 (Acetonitril) | analog Beispiel 2 |
| 22 | Cl | -NH- | 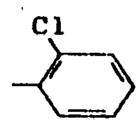 | 151 (Z.) | analog Beispiel 1 |

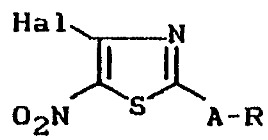

(I)

| Verbin-dungs-Nummer | Hal | - A - | - R | Schmp. (°C) (umkrist. aus) | Herstellung |
|---|---|---|---|---|---|
| 23 | Cl | -NH- | 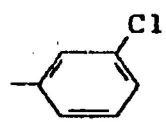 | 197 (Z.) | analog Beispiel 1 |
| 24 | Cl | -NH- | 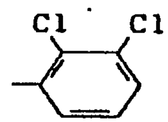 | 108 - 11 | analog Beispiel 1 |
| 25 | Cl | -NH- | 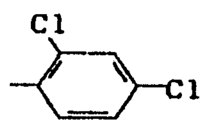 | 188 - 9 (Z.) | analog Beispiel 1 |
| 26 | Cl | -NH- | 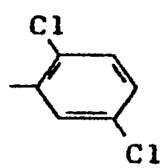 | 136 - 8 (Z.) | analog Beispiel 1 |
| 27 | Cl | -NH- | 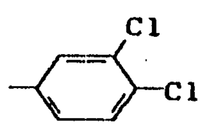 | 214 - 5 (Z.) | analog Beispiel 1 |

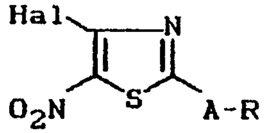

(I)

| Verbin-dungs-Nummer | Hal | - A - | - R | Schmp. (°C) (umkrist. aus) | Herstellung |
|---|---|---|---|---|---|
| 28 | Cl | -NH- | 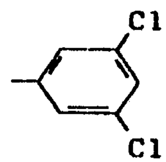 | 142 - 4 (Z.) | analog Beispiel 1 |
| 29 | Cl | -N- 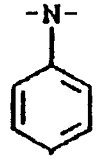 | 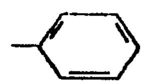 | 171 - 3 (Z.) | analog Beispiel 10 |
| 30 | Cl | -NH- | 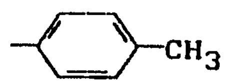 | 194 (Z.) | analog Beispiel 1 |
| 31 | Cl | -NH- | 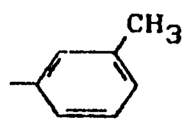 | 175 (Z.) | analog Beispiel 1 |
| 32 | Cl | -NH- | 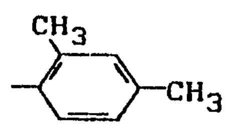 | 177 - 8 (Z.) | analog Beispiel 1 |

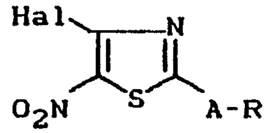

(I)

| Verbin-dungs-Nummer | Hal | - A - | - R | Schmp. (°C) (umkrist. aus) | Herstellung |
|---|---|---|---|---|---|
| 33 | Cl | -NH- | 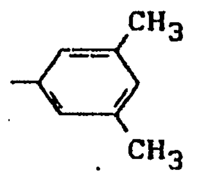 | 179 - 80 (Z.) | analog Beispiel 1 |
| 34 | Cl | -NH- | 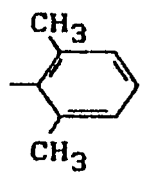 | 215 - 6 (Z.) | analog Beispiel 1 |
| 35 | Cl | -NH- | 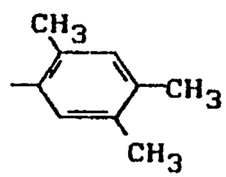 | 183 - 5 (Z.) | analog Beispiel 1 |
| 36 | Cl | -NH- | 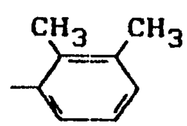 | 202 (Z.) | analog Beispiel 1 |
| 37 | Cl | -N-<br>$CH_3$ | 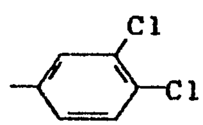 | 123 - 4 | analog Beispiel 1 |

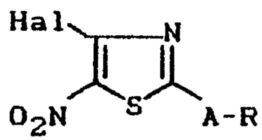 (I)

| Verbin-dungs-Nummer | Hal | - A - | - R | Schmp. (°C) (umkrist. aus) | Herstellung |
|---|---|---|---|---|---|
| 38 | Cl | -NH- | 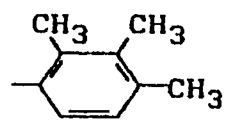 | 216 - 7 (Z.) | analog Beispiel 1 |
| 39 | Cl | -NH- | 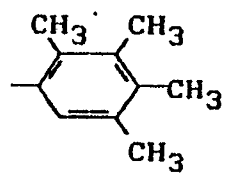 | 170 (Z.) | analog Beispiel 1 |
| 40 | Cl | -NH- | 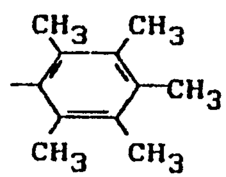 | 243 - 4 (Z.) | analog Beispiel 1 |
| 41 | Cl | -NH- | 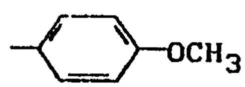 | 193 (Z.) | analog Beispiel 4 |
| 42 | I | -NH- | 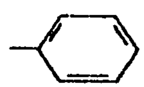 | 168 - 9 (Z.) (Cyclohexan) | analog Beispiel 1 (aus 2,4-Diiod-5-nitrothiazol) |

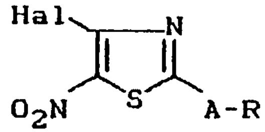

(I)

| Verbin-dungs-Nummer | Hal | - A - | - R | Schmp. (°C) (umkrist. aus) | Herstellung |
|---|---|---|---|---|---|
| 43 | Cl | -NH- | 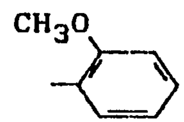 | 185 - 6 (Z.) | analog Beispiel 1 |
| 44 | Cl | -NH- | 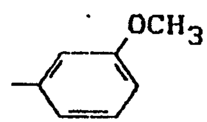 | 175 - 6 (Z.) | analog Beispiel 1 |
| 45 | Cl | -O- | $-CH_3$ | 52 (Petrolether) | s. Beispiel 9 |
| 46 | Cl | -NH- | 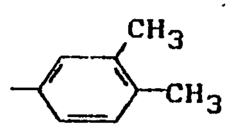 | 178 - 9 (Z.) | analog Beispiel 1 |
| 47 | Cl | -NH- | 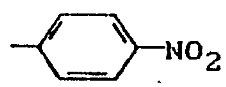 | 262 - 3 (Z.) | analog Beispiel 1 |
| 48 | Cl | $-N(C_2H_5)-$ | 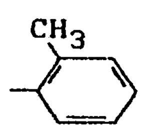 | Öl | analog Beispiel 1 |

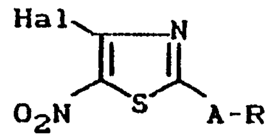

(I)

| Verbin-dungs-Nummer | Hal | - A - | - R | Schmp. (°C) (umkrist. aus) | Herstellung |
|---|---|---|---|---|---|
| 49 | Cl | -NH- | 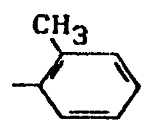 | 180 - 1 (Z.) | analog Beispiel 1 |
| 50 | Cl | -NH- | 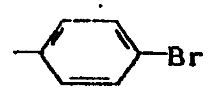 | 208 (Z.) | analog Beispiel 1 |
| 51 | Cl | -NH- | 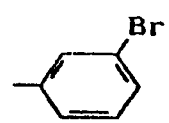 | 191 - 2 (Z.) | s. Beispiel 1 |
| 52 | Cl | -O- | 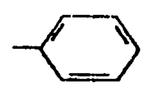 | 96 (Petrolether) | s. Beispiel 2 |
| 53 | Cl | -O- | 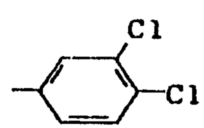 | 83 - 4 | analog Beispiel 2 |
| 54 | Cl | -O- | 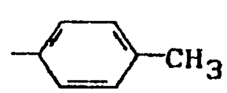 | 94 - 6 | analog Beispiel 2 |

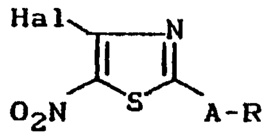 (I)

| Verbindungs-Nummer | Hal | - A - | - R | Schmp. (°C) (umkrist. aus) | Herstellung |
|---|---|---|---|---|---|
| 55 | Cl | -O- | 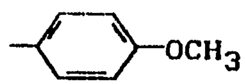 | 107 - 8 | analog Beispiel 2 |
| 56 | Cl | -S- | 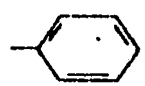 | 87 - 9 | analog Beispiel 2 |
| 57 | Cl | -S- | 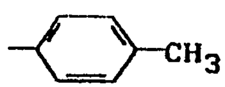 | 83 - 5 | analog Beispiel 2 |
| 58 | Cl | -S- | 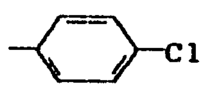 | 93 | analog Beispiel 2 |
| 59 | Cl | 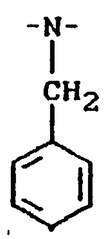 | 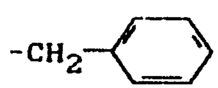 | 94 (Cyclohexan) | analog Beispiel 2 |

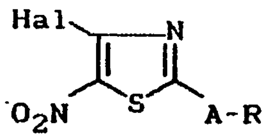

(I)

| Verbindungs-Nummer | Hal | - A - | - R | Schmp. (°C) (umkrist. aus) | Herstellung |
|---|---|---|---|---|---|
| 60 | Cl | -N-<br>$C_2H_5$ | 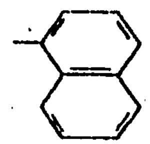 | 240 (Z.) | analog Beispiel 2 |
| 61 | Cl | -NH- | 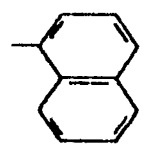 | 178 - 9 (Z.) | analog Beispiel 1 |
| 62 | Cl | -NH- | 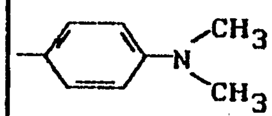 | › 260 | analog Beispiel 1 |
| 63 | Cl | -NH- | 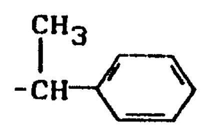 | 108 (Cyclohexan) | analog Beispiel 4 |
| 64 | Cl | -S- | 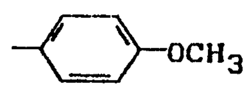 | 62 - 5 | analog Beispiel 2 |

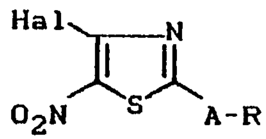

(I)

| Verbin-dungs-Nummer | Hal | - A - | - R | Schmp. (°C) (umkrist. aus) | Herstellung |
|---|---|---|---|---|---|
| 65 | Cl | -N-<br>$CH_3$ | $-CH_3$ | 182,5 | analog Beispiel 3 |
| 66 | Cl | $-SO_2-$ | 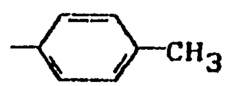 | 153 - 153,5 (Cyclohexan) | s. Beispiele 12 u. 13 |
| 67 | Cl | -A-R = | 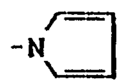 | 138 - 9 (Toluol) | s. Beispiel 10 |
| 68 | Cl | -NH- | $-CH_3$ | 212 (Z.) (Methanol) | s. Beispiel 11 |
| 69 | Cl | -NH- | 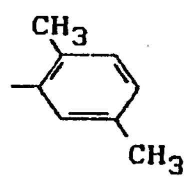 | 186 - 7 (Z.) | analog Beispiel 1 |
| 70 | Cl | -NH- | 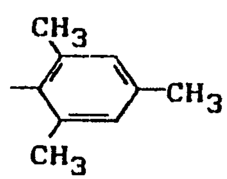 | 214 - 5 (Z.) | analog Beispiel 1 |

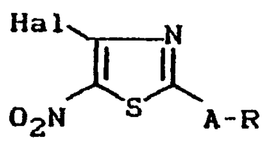 (I)

| Verbindungs-Nummer | Hal | - A - | - R | Schmp. (°C) (umkrist. aus) | Herstellung |
|---|---|---|---|---|---|
| 71 | Cl | -NH- | 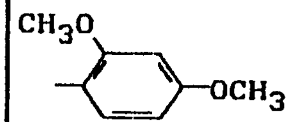 | 167 - 9 (Z.) | analog Beispiel 1 |
| 72 | Cl | -NH- | 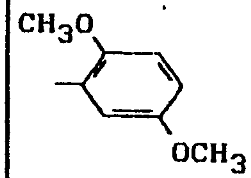 | 153 - 4 (Z.) | analog Beispiel 1 |
| 73 | Cl | -SO- | 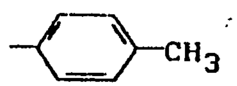 | 112 | s. Beispiel 14 |
| 74 | Cl | -NH- | 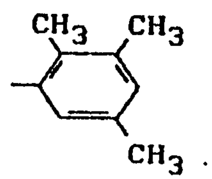 | 208 - 9 (Z.) | analog Beispiel 1 |
| 75 | Cl | -O- | 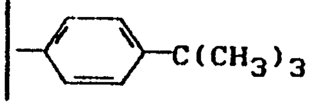 | 87 - 8 | analog Beispiel 2 |

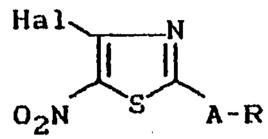

(I)

| Verbindungs-Nummer | Hal | - A - | - R | Schmp. (°C) (umkrist. aus) | Herstellung |
|---|---|---|---|---|---|
| 76 | Cl | -O- | 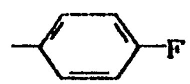 | 103 - 4 | analog Beispiel 2 |
| 77 | Cl | -O- | 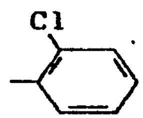 | 79 - 80 | analog Beispiel 2 |
| 78 | Cl | -O- | 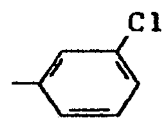 | 43 | analog Beispiel 2 |
| 79 | Cl | -O- | 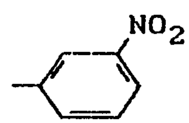 | 106 - 7 | analog Beispiel 2 |
| 80 | Cl | -O- | 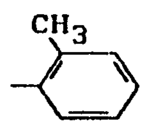 | Öl | analog Beispiel 2 |
| 81 | Cl | -O- | 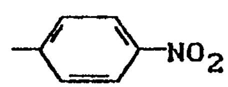 | 147 - 8 | analog Beispiel 2 |

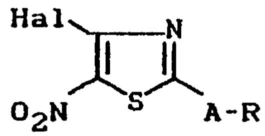 (I)

| Verbin-dungs-Nummer | Hal | - A - | - R | Schmp. (°C) (umkrist. aus) | Herstellung |
|---|---|---|---|---|---|
| 82 | Cl | -O- | 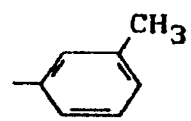 | 44 - 5 | analog Beispiel 2 |
| 83 | Cl | -O- | 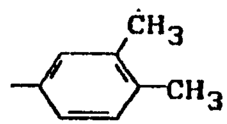 | 76 - 7 | analog Beispiel 2 |
| 84 | Cl | -S- | 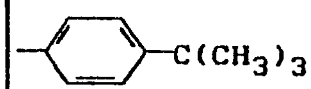 | Öl | analog Beispiel 2 |
| 85 | Cl | -O- | 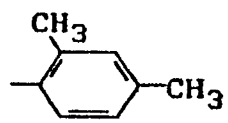 | 54 | analog Beispiel 2 |
| 86 | Cl | -O- | 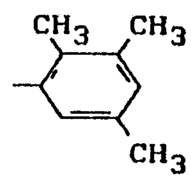 | 98 | analog Beispiel 2 |

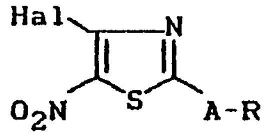

(I)

| Verbin-dungs-Nummer | Hal | - A - | - R | Schmp. (°C) (umkrist. aus) | Herstellung |
|---|---|---|---|---|---|
| 87 | Cl | -O- | 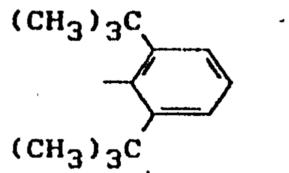 | 188 - 9 | analog Beispiel 2 |
| 88 | Cl | -NH- | 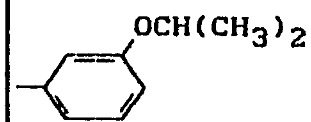 | 48 - 9 | analog Beispiel 1 |
| 89 | Cl | -O- | 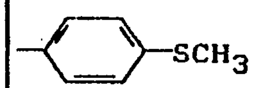 | 96 | analog Beispiel 2 |
| 90 | Cl | -O- | 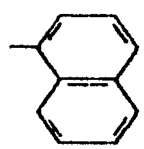 | 51 - 2 | analog Beispiel 2 |
| 91 | Cl | -O- | 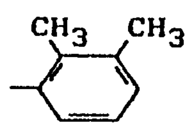 | 45 | analog Beispiel 2 |

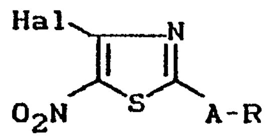

(I)

| Verbin-dungs-Nummer | Hal | - A - | - R | Schmp. (°C) (umkrist. aus) | Herstellung |
|---|---|---|---|---|---|
| 92 | Cl | -O- | 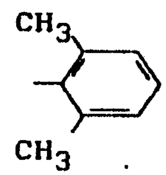 | Öl | analog Beispiel 2 |
| 93 | Cl | -O- | 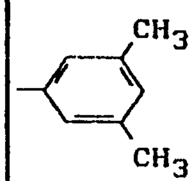 | 57 | analog Beispiel 2 |
| 94 | Cl | -O- | 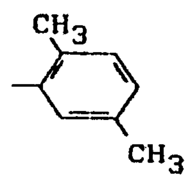 | 61 | analog Beispiel 2 |
| 95 | Cl | -NH- | 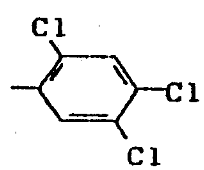 | 191 - 2 (Z.) | analog Beispiel 1 |
| 96 | Cl | -NH- | 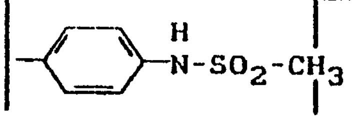 | 203 (Z.) | analog Beispiel 1 |

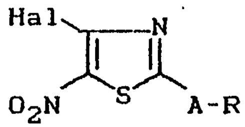

(I)

| Verbindungs-Nummer | Hal | - A - | - R | Schmp. (°C) | Herstellung |
|---|---|---|---|---|---|
| 153 | Cl | -O- | 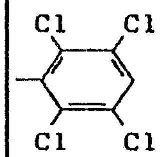 | 208-9 | analog Beispiel 2 |
| 154 | Cl | -O- | 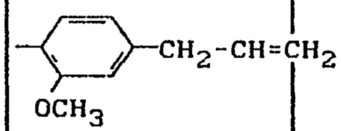 | Öl | analog Beispiel 2 |
| 155 | Cl | -O- | 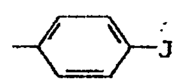 | 98 | analog Beispiel 2 |
| 156 | Cl | -O- | 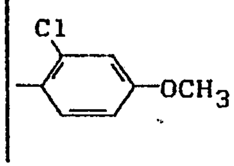 | 83 | analog Beispiel 2 |

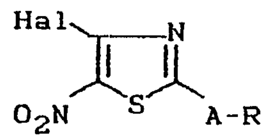

(I)

| Verbin-dungs-Nummer | Hal | - A - | - R | Schmp. (°C) (umkrist. aus) | Herstellung |
|---|---|---|---|---|---|
| 103 | Cl | -NH- | 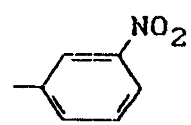 | 163 - 4 (Z.) | analog Beispiel 1 |
| 104 | Cl | -O- | 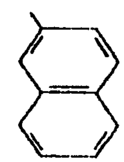 | 128 | analog Beispiel 2 |
| 105 | Cl | -O- | 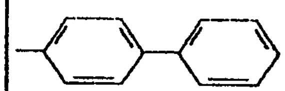 | 187 | analog Beispiel 2 |
| 106 | Cl | -S- | 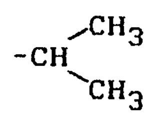 | Öl | analog Beispiel 15 |
| 107 | Cl | -S- | $-C(CH_3)_3$ | Öl | siehe Beispiel 15 |
| 108 | Cl | -S- | 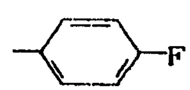 | 95 | analog Beispiel 2 |

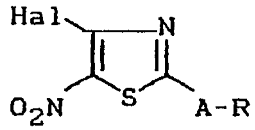

(I)

| Verbin-dungs-Nummer | Hal | - A - | - R | Schmp. (°C) (umkrist. aus) | Herstellung |
|---|---|---|---|---|---|
| 109 | Cl | -O- | 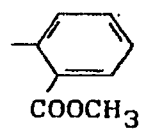 | 56 | analog Beispiel 2 |
| 110 | Cl | -O- | 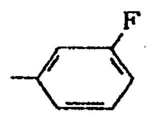 | 67 | analog Beispiel 2 |
| 111 | I | -N-<br>$CH_3$ | 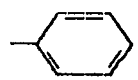 | 86 - 8 (Z.) | analog Beispiel 2<br>aus 2-Chlor-4-iod-5-nitrothiazol |
| 112 | Br | -N-<br>$CH_3$ | 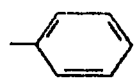 | 102 - 4 (Z.) | analog Beispiel 1 |
| 113 | Cl | -N-<br>H | $-CH_2-CH = CH_2$ | 130 | analog Beispiel 1 |
| 114 | Cl | -N-<br>H | 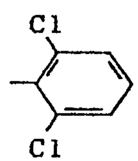 | 141<br>(Petrolether) | in Tetrahydrofuran<br>unter Zusatz von Natriumhydrid |

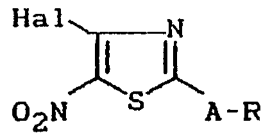

(I)

| Verbin-dungs-Nummer | Hal | - A - | - R | Schmp. (°C) | Herstellung |
|---|---|---|---|---|---|
| 157 | Cl | -O- | 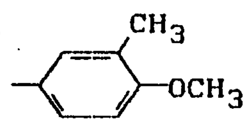 | 96 | analog Beispiel 2 |
| 158 | Cl | -NH- | 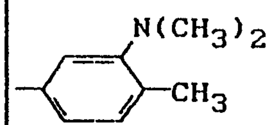 | 106-7 (Z.) | analog Beispiel 1 |
| 159 | Cl | -O- | 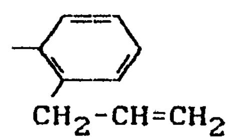 | Öl | analog Beispiel 2 |
| 160 | Cl | -O- | 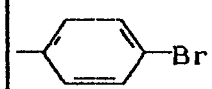 | 97 | analog Beispiel 2 |

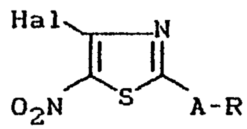

(I)

| Verbin-<br>dungs-<br>Nummer | Hal | - A - | - R | Schmp. (°C)<br>(umkrist. aus) | Herstellung |
|---|---|---|---|---|---|
| 121 | Cl | -N-<br>H | 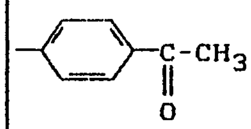 | 262 - 3 (Z.) | analog Beispiel 1 |
| 122 | Cl | -N-<br>H | 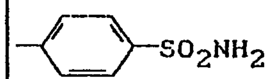 | ›280 | analog Beispiel 1 |
| 123 | Cl | -N-<br>H | 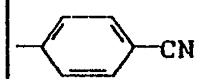 | 266 - 7 (Z.) | analog Beispiel 1 |
| 124 | Cl | -N-<br>H | 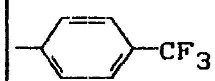 | 202 - 3 (Z.) | analog Beispiel 1 |
| 125 | Cl | -N-<br>H | 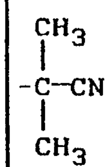 | 127 - 8 | analog Beispiel 1 |
| 126 | Cl | 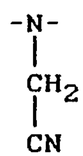 | $-CH_2-CH_2-CN$ | 176 (Z.) | analog Beispiel 1 |

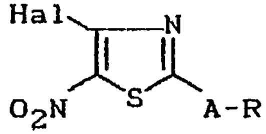

(I)

| Verbin-dungs-Nummer | Hal | - A - | - R | Schmp. (°C) (umkrist. aus) | Herstellung |
|---|---|---|---|---|---|
| 127 | Cl | -N- H | 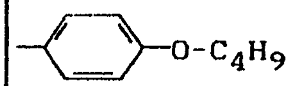 | 154 - 5 (Z.) | analog Beispiel 1 |
| 128 | Cl | -N- H | 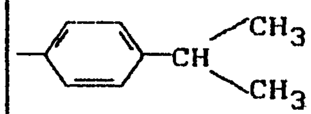 | 161 - 2 (Z.) | analog Beispiel 1 |
| 129 | Cl | -O- | 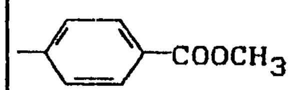 | 124 - 5 | analog Beispiel 2 |
| 130 | Cl | -O- | 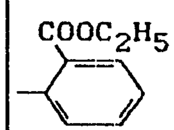 | 111 - 2 | analog Beispiel 2 |
| 131 | Cl | -N- H | 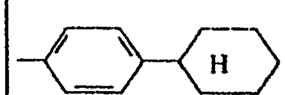 | 185 - 6 (Z.) | analog Beispiel 1 |
| 132 | Cl | -N- H | 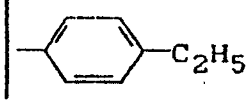 | 174 - 5 (Z.) | analog Beispiel 1 |

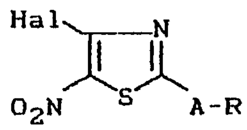

(I)

| Verbin-dungs-Nummer | Hal | - A - | - R | Schmp. (°C) (umkrist. aus) | Herstellung |
|---|---|---|---|---|---|
| 133 | Cl | -N-<br>H | 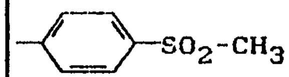 | 268 - 9 (Z.) | analog Beispiel 1 |
| 134 | Cl | -N-<br>H | 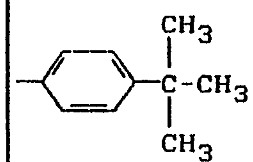 | 189 - 90 (Z.) | analog Beispiel 1 |
| 135 | Cl | -O- | 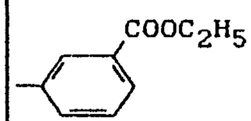 | 80 | analog Beispiel 2 |
| 136 | Cl | -N-<br>H | 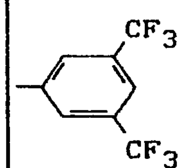 | 151 - 2 (Z.) | analog Beispiel 1 |
| 137 | Cl | -N-<br>H | 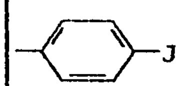 | 165 - 6 (Z.) | analog Beispiel 1 |
| 138 | Cl | -N-<br>H | 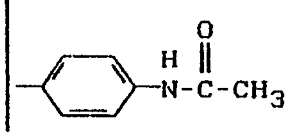 | 226 - 7 (Z.) | analog Beispiel 1 |

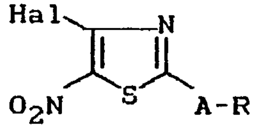 (I)

| Verbin-dungs-Nummer | Hal | - A - | - R | Schmp. (°C) (umkrist. aus) | Herstellung |
|---|---|---|---|---|---|
| 139 | Cl | -N- H | 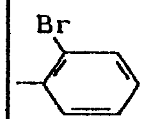 | 157 - 8 (Z.) | analog Beispiel 1 |
| 140 | Cl | -S- | 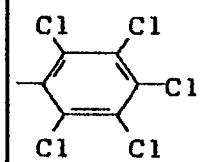 | 154 - 5 (Z.) (Acetonitril) | analog Beispiel 2 |
| 141 | Cl | -N- H | 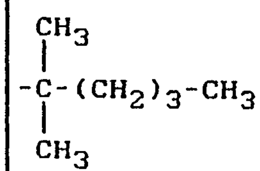 | Öl | analog Beispiel 1 |
| 142 | Cl | -S- | 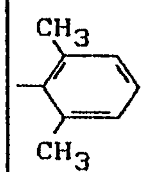 | 95 | analog Beispiel 2 |
| 143 | Cl | -S- | 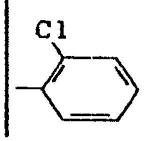 | 52 - 3 | analog Beispiel 2 |

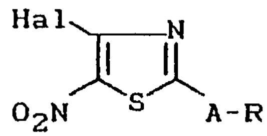 (I)

| Verbin-dungs-Nummer | Hal | - A - | - R | Schmp. (°C) (umkrist. aus) | Herstellung |
|---|---|---|---|---|---|
| 144 | Cl | -S- | 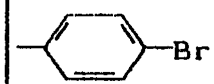 | 80 - 1 | analog Beispiel 2 |
| 145 | Cl | -O- | 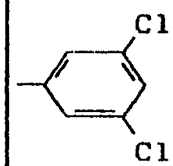 | 123 - 4 | analog Beispiel 2 |
| 146 | Cl | -O- | 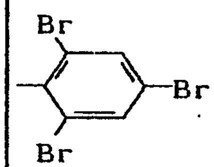 | 134 - 5 | analog Beispiel 2 |
| 147 | Cl | -O- | 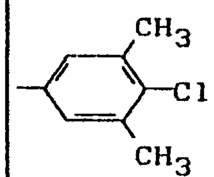 | 75 - 6 | analog Beispiel 2 |
| 148 | Cl | -S- | 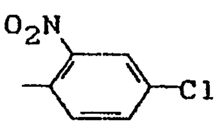 | 117 - 8 | analog Beispiel 2 |

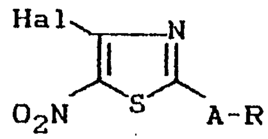

(I)

| Verbin-dungs-Nummer | Hal | - A - | - R | Schmp. (°C) (umkrist. aus) | Herstellung |
|---|---|---|---|---|---|
| 149 | Cl | -S- | 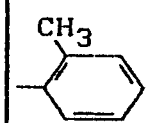 | 96-7 | analog Beispiel 2 |
| 150 | Cl | -O- | 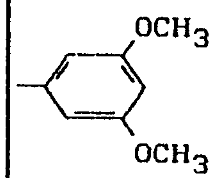 | Öl | analog Beispiel 2 |
| 151 | Cl | -O- | 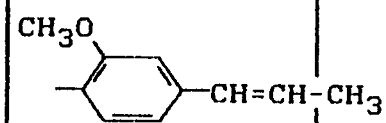 | 57 | analog Beispiel 2 |
| 152 | Cl | -O- | 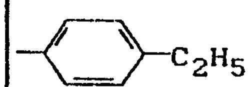 | 58 - 9 | analog Beispiel 2 |

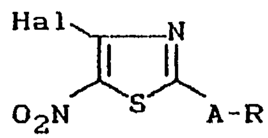

(I)

| Verbin-dungs-Nummer | Hal | - A - | - R | Schmp. (°C) | Herstellung |
|---|---|---|---|---|---|
| 153 | Cl | -O- | 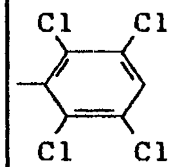 | 208-9 | analog Beispiel 2 |
| 154 | Cl | -O- | 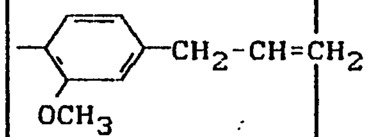 | Öl | analog Beispiel 2 |
| 155 | Cl | -O- | 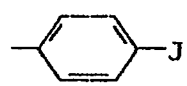 | 98 | analog Beispiel 2 |
| 156 | Cl | -O- | 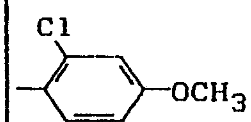 | 83 | analog Beispiel 2 |

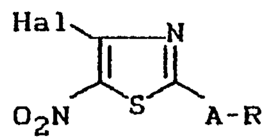 (I)

| Verbindungs-Nummer | Hal | - A - | - R | Schmp. (°C) | Herstellung |
|---|---|---|---|---|---|
| 157 | Cl | -O- | 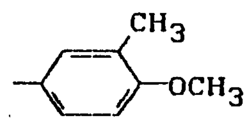 | 96 | analog Beispiel 2 |
| 158 | Cl | -NH- | 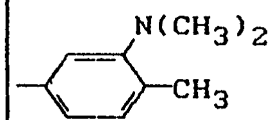 | 106-7 (Z.) | analog Beispiel 1 |
| 159 | Cl | -O- | 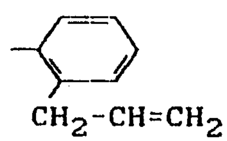 | Öl | analog Beispiel 2 |
| 160 | Cl | -O- | 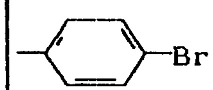 | 97 | analog Beispiel 2 |

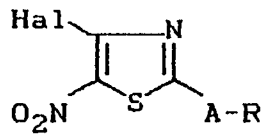

(I)

| Verbin-dungs-Nummer | Hal | - A - | - R | Schmp. (°C) | Herstellung |
|---|---|---|---|---|---|
| 161 | Cl | -O- | 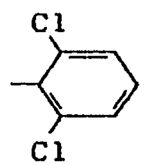 | 106 | analog Beispiel 2 |
| 162 | Cl | -O- | 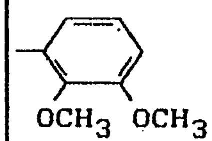 | 116 | analog Beispiel 2 |
| 163 | Cl | -O- | 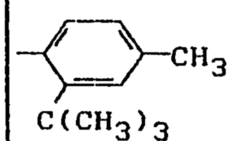 | Öl | analog Beispiel 2 |
| 164 | Cl | -O- | 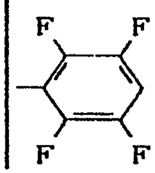 | 68 | analog Beispiel 2 |

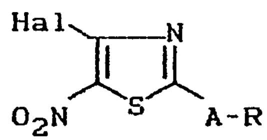

(I)

| Verbin-dungs-Nummer | Hal | - A - | - R | Schmp. (°C) | Herstellung |
|---|---|---|---|---|---|
| 165 | Cl | -O- | 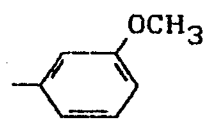 | 78 | analog Beispiel 2 |
| 166 | Cl | -O- | 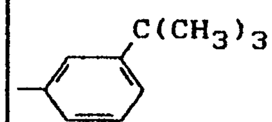 | 68 | analog Beispiel 2 |
| 167 | Cl | -O- | 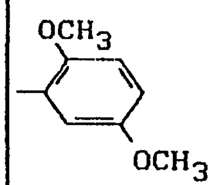 | 115 | analog Beispiel 2 |
| 168 | Cl | -O- | 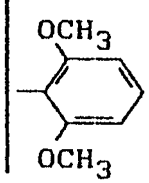 | 152 | analog Beispiel 2 |

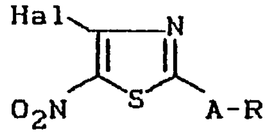

(I)

| Verbin-dungs-Nummer | Hal | - A - | - R | Schmp. (° C) | Herstellung |
|---|---|---|---|---|---|
| 169 | Cl | -O- | 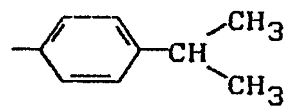 | 86 | analog Beispiel 2 |
| 170 | Cl | -O- | 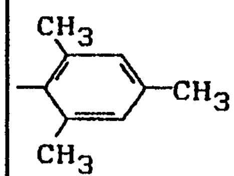 | Öl | analog Beispiel 2 |
| 171 | Cl | -O- | 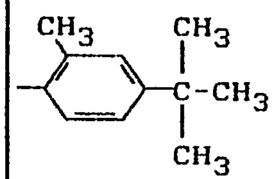 | Öl | analog Beispiel 2 |
| 172 | Cl | -O- | 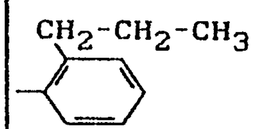 | Öl | analog Beispiel 2 |

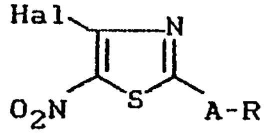 (I)

| Verbin-dungs-Nummer | Hal | - A - | - R | Schmp. (°C) | Herstellung |
|---|---|---|---|---|---|
| 173 | Cl | -O- | 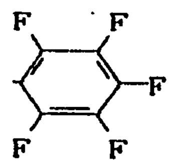 | 52 | analog Beispiel 2 |
| 174 | Cl | $-SO_2-$ | 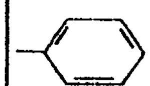 | 125 (Zers.) | analog Beispiel 13 |
| 175 | Cl | -O- | 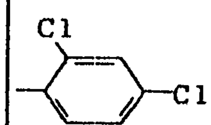 | Öl | analog Beispiel 2 |
| 176 | Cl | -O- | 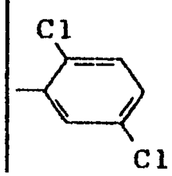 | 122 | analog Beispiel 2 |

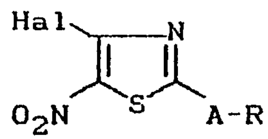

(I)

| Verbin-dungs-Nummer | Hal | - A - | - R | Schmp. (°C) | Herstellung |
|---|---|---|---|---|---|
| 177 | Cl | -O- | 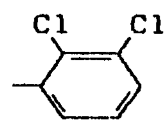 | 113 | analog Beispiel 2 |
| 178 | Cl | -O- | 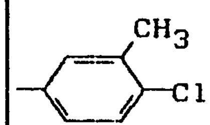 | 86 | analog Beispiel 2 |
| 179 | Cl | -O- | 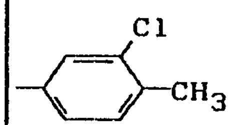 | Öl | analog Beispiel 2 |
| 180 | Cl | H<br>-N- | 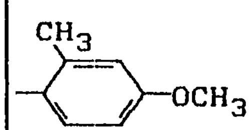 | 144 (Z.) | analog Beispiel 1 |

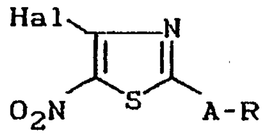

(I)

| Verbin-dungs-Nummer | Hal | - A - | - R | Schmp. (°C) | Herstellung |
|---|---|---|---|---|---|
| 181 | Cl | H<br>-N- | 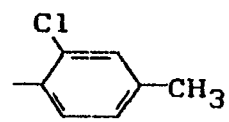 | 162-3 (Z.) | analog Beispiel 1 |
| 182 | Cl | H<br>-N- | 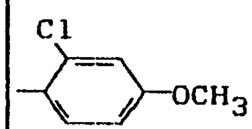 | 148 (Z.) | analog Beispiel 1 |
| 183 | Cl | H<br>-N- | 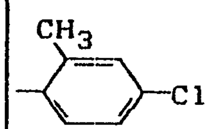 | 201-2 (Z.) | analog Beispiel 1 |
| 184 | Cl | H<br>-N- | 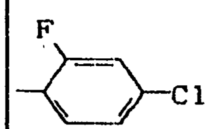 | 205-6 (Z.) | analog Beispiel 1 |

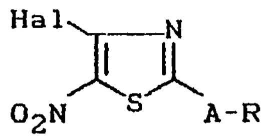

(I)

| Verbin-<br>dungs-<br>Nummer | Hal | - A - | - R | Schmp. (°C) | Herstellung |
|---|---|---|---|---|---|
| 185 | Cl | H<br>-N- | 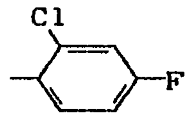 | 168-9 (Z.) | analog Beispiel 1 |
| 186 | Cl | H<br>-N- | 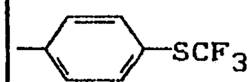 | 126 (Z.) | analog Beispiel 1 |
| 187 | Cl | H<br>-N- | 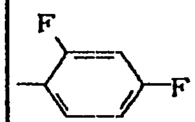 | 117 (Z.) | analog Beispiel 1 |
| 188 | Cl | -N-<br>$CH_3$ | 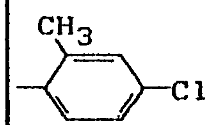 | 140-1 (Z.) | analog Beispiel 1 |

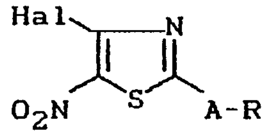

(I)

| Verbin-dungs-Nummer | Hal | - A - | - R | Schmp. (°C) | Herstellung |
|---|---|---|---|---|---|
| 189 | Cl | H<br>-N- | 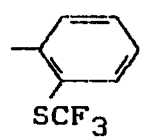 | 126-7 (Z.) | analog Beispiel 1 |
| 190 | Cl | 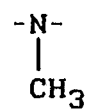 | 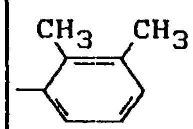 | 179-80 (Z.) | analog Beispiel 1 |
| 191 | Cl | H<br>-N- | 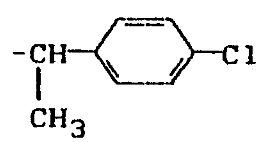 | Öl | analog Beispiel 1 |
| 192 | Cl | H<br>-N- | 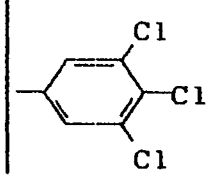 | 221 (Z.) | analog Beispiel 1 |

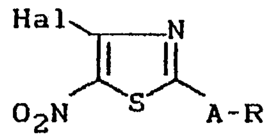

(I)

| Verbin-dungs-Nummer | Hal | - A - | - R | Schmp. ($^{o}C$) | Herstellung |
|---|---|---|---|---|---|
| 193 | Cl | H<br>-N- | 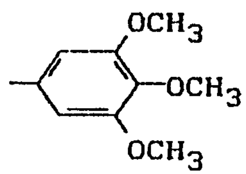 | 177-8 (Z.) | analog Beispiel 1 |
| 194 | Cl | H<br>-N- | 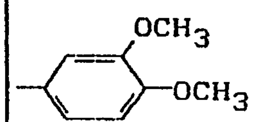 | 201 (Z.) | analog Beispiel 1 |
| 195 | Cl | $CH_3$<br>-N- | 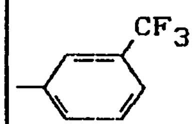 | 94-5 | analog Beispiel 1 |
| 196 | Cl | $C_2H_5$<br>-N- | 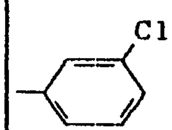 | 87-8 | analog Beispiel 1 |

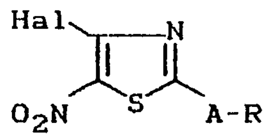

(I)

| Verbin-dungs-Nummer | Hal | - A - | - R | Schmp. (°C) | Herstellung |
|---|---|---|---|---|---|
| 197 | Cl | $C_2H_5$<br>-N- | 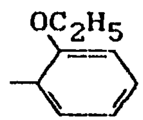 | 71 | analog Beispiel 1 |
| 198 | Cl | $CH_3$<br>-N- | 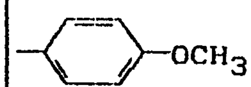 | 141 (Z.) | analog Beispiel 1 |
| 199 | Cl | $C_2H_5$<br>-N- | 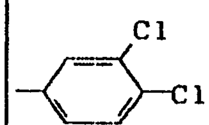 | 144 (Z.) | analog Beispiel 1 |
| 200 | Cl | H<br>-N- | 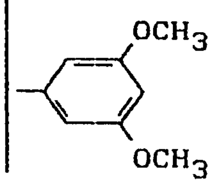 | 97-8 (Z.) | analog Beispiel 1 |

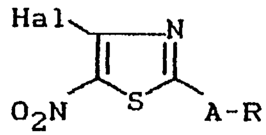

(I)

| Verbin-dungs-Nummer | Hal | - A - | - R | Schmp. (°C) | Herstellung |
|---|---|---|---|---|---|
| 201 | Cl | H<br>-N- | 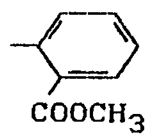 | 161-2 (Z.) | analog Beispiel 1 |
| 202 | Cl | H<br>-N- | 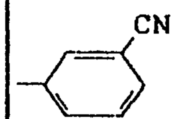 | 224-5 (Z.) | analog Beispiel 1 |
| 203 | Cl | H<br>-N- | 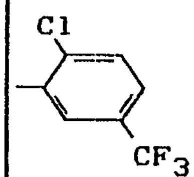 | 159-60 (Z.) | analog Beispiel 1 |
| 204 | Cl | H<br>-N- | 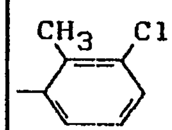 | 204-5 (Z.) | analog Beispiel 1 |

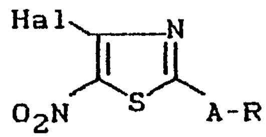

(I)

| Verbin-dungs-Nummer | Hal | - A - | - R | Schmp. (°C) | Herstellung |
|---|---|---|---|---|---|
| 205 | Cl | H<br>-N- | 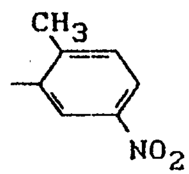 | 213-4 (Z.) | analog Beispiel 1 |
| 206 | Cl | H<br>-N- | 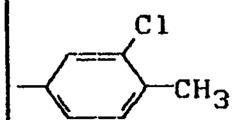 | 161-2 (Z.) | analog Beispiel 1 |
| 207 | Cl | H<br>-N- | 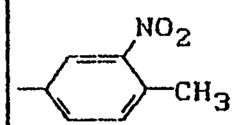 | 209 (Z.) | analog Beispiel 1 |
| 208 | Cl | -A-R= -N 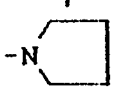 | | 162-3 (Z.) | analog Beispiel 3 |

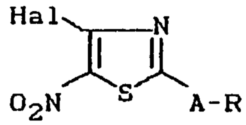

(I)

| Verbin-dungs-Nummer | Hal | - A - | - R | Schmp. (°C) | Herstellung |
|---|---|---|---|---|---|
| 209 | Cl | -A-R=- 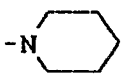 | | 91-2 | analog Beispiel 3 |
| 210 | Cl | -A-R=- 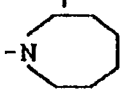 | | 113 (Z.) | analog Beispiel 3 |
| 211 | Cl | H -N- | 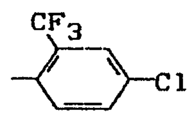 | 167-8 (Z.) | analog Beispiel 1 |
| 212 | Cl | H -N- | 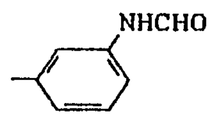 | 256 (Z.) | analog Beispiel 1 |
| 213 | Cl | H -N- | 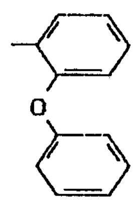 | 83 | analog Beispiel 1 |

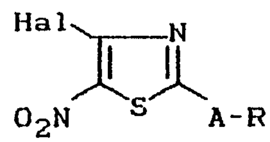

(I)

| Verbin-<br>dungs-<br>Nummer | Hal | - A - | - R | Schmp. (°C) | Herstellung |
|---|---|---|---|---|---|
| 214 | Cl | -N-<br>$CH_3$ | 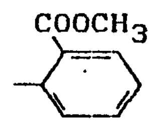 | 112-3 | analog Beispiel 1 |
| 215 | Cl | H<br>-N- | 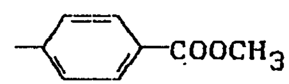 | 254 (Z.) | analog Beispiel 1 |
| 216 | Cl | H<br>-N- | 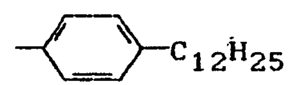 | Öl | analog Beispiel 1 |
| 217 | Cl | H<br>-N- | 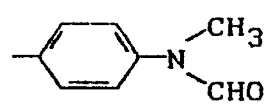 | 221 (Z.) | analog Beispiel 1 |
| 218 | Cl | H<br>-N- | 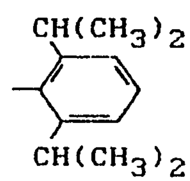 | 228 (Z.) | analog Beispiel 1 |

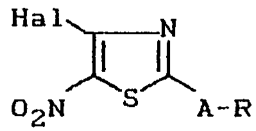 (I)

| Verbin-dungs-Nummer | Hal | - A - | - R | Schmp. (°C) | Herstellung |
|---|---|---|---|---|---|
| 219 | Cl | H<br>-N- | 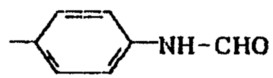 | 168-9 (Z.) | analog Beispiel 1 |
| 220 | Cl | H<br>-N- | 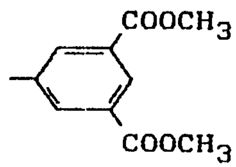 | 117-8 (Z.) | analog Beispiel 1 |
| 221 | Cl | H<br>-N- | 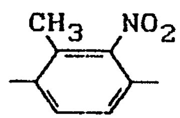 | 156-7 (Z.) | analog Beispiel 1 |
| 222 | Cl | H<br>-N- | 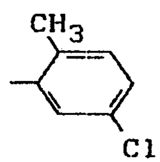 | 202 (Z.) | analog Beispiel 1 |
| 223 | Cl | H<br>-N- | 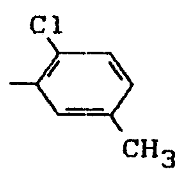 | 186-7 (Z.) | analog Beispiel 1 |

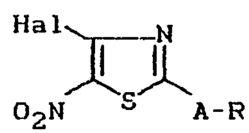

(I)

| Verbin-dungs-Nummer | Hal | - A - | - R | Schmp. (°C) | Herstellung |
|---|---|---|---|---|---|
| 224 | Cl | H<br>-N- | 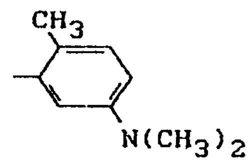 | 163-4 (Z.) | analog Beispiel 1 |
| 225 | Cl | H<br>-N- | 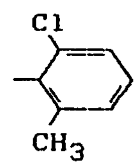 | 156-7 (Z.) | analog Beispiel 1 |
| 226 | Cl | H<br>-N- | 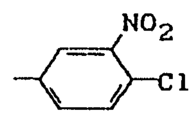 | 148-9 (Z.) | analog Beispiel 1 |
| 227 | Cl | H<br>-N- | 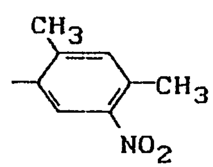 | 230 (Z.) | analog Beispiel 1 |
| 228 | Cl | H<br>-N- | 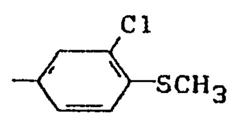 | 159-60 (Z. | analog Beispiel 1 |

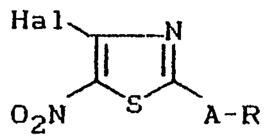

(I)

| Verbin-dungs-Nummer | Hal | - A - | - R | Schmp. ($^oC$) | Herstellung |
|---|---|---|---|---|---|
| 229 | Cl | -O- | 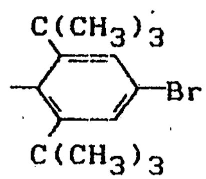 | 247 | analog Beispiel 2 |
| 230 | Cl | H<br>-N- | 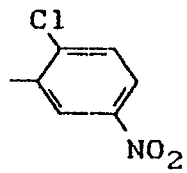 | 198-9 (Z.) | analog Beispiel 1 |
| 231 | Cl | $C_2H_5$<br>\|<br>-N- | 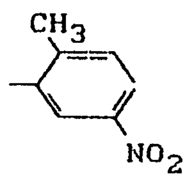 | 149-50 (Z.) | analog Beispiel 1 |
| 232 | Cl | H<br>-N- | $-C_2H_5$ | 167-8 (Z.) | analog Beispiel 11 |
| 233 | Cl | H<br>-N- | $-CH_2-C_2H_5$ | 106 (Z.) | analog Beispiel 11 |

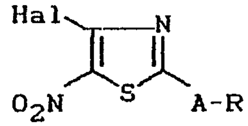

(I)

| Verbin-dungs-Nummer | Hal | - A - | - R | Schmp. (°C) | Herstellung |
|---|---|---|---|---|---|
| 234 | Cl | H<br>-N- | 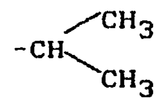 | 125-7 (Z.) | analog Beispiel 11 |
| 235 | Cl | H<br>-N- | $-CH_2-CH_2-OCH_3$ | 124-8 | analog Beispiel 11 |
| 236 | Cl | H<br>-N- | $-CH_2-CH_2-Cl$ | 143-4 (Z.) | analog Beispiel 11 |
| 237 | Cl | -S- | 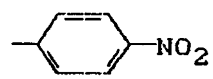 | 97-8 (Z.) | analog Beispiel 2 |
| 238 | Cl | -O- | 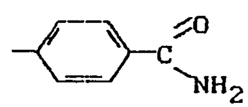 | 136-7 (Z.) | analog Beispiel 2 |
| 239 | Cl | -O- | 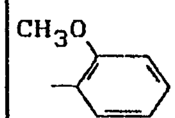 | 186-7 (Z.) | analog Beispiel 2 |
| 240 | Cl | -O- | 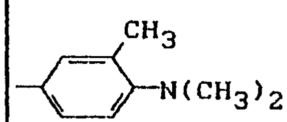 | 82 | analog Beispiel 2 |

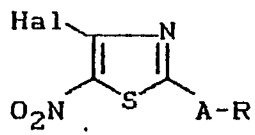 (I)

| Verbindungs-Nummer | Hal | - A - | - R | Schmp. (°C) | Herstellung |
|---|---|---|---|---|---|
| 241 | Cl | -O- | 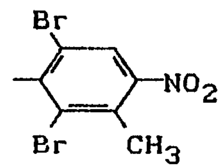 | 142 | analog Beispiel 2 |
| 242 | Cl | -O- | 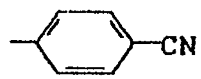 | 103-4 | analog Beispiel 2 |
| 243 | Cl | -O- | 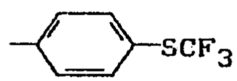 | 71 | analog Beispiel 2 |
| 244 | Cl | -O- | 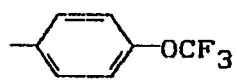 | 48 | analog Beispiel 2 |
| 245 | Cl | -O- | 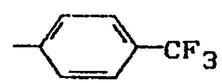 | 77 | analog Beispiel 2 |
| 246 | Cl | -O- | 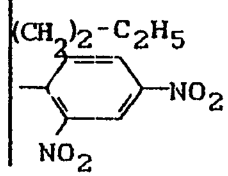 | Öl | analog Beispiel 2 |

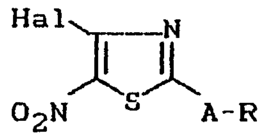

(I)

| Verbin-dungs-Nummer | Hal | - A - | - R | Schmp. (°C) | Herstellung |
|---|---|---|---|---|---|
| 247 | Cl | -O- | 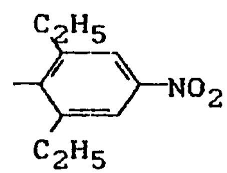 | 142 (Z.) | analog Beispiel 2 |
| 248 | Cl | -O- | 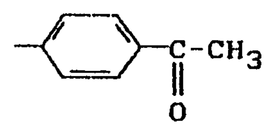 | 108 | analog Beispiel 2 |
| 249 | Cl | -O- | 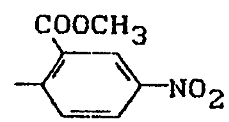 | 102-3 | analog Beispiel 2 |
| 250 | Cl | -O- | 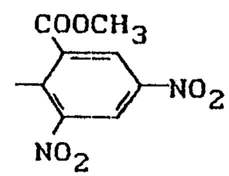 | 104 | analog Beispiel 2 |
| 251 | Cl | -O- | 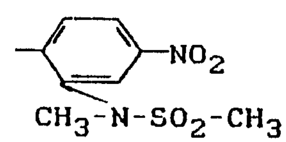 | 159 | analog Beispiel 2 |

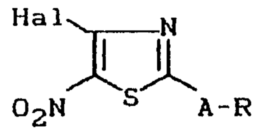

(I)

| Verbin-dungs-Nummer | Hal | - A - | - R | Schmp. (°C) | Herstellung |
|---|---|---|---|---|---|
| 252 | Cl | -O- | 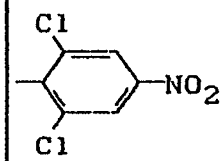 | 145 | analog Beispiel 2 |
| 253 | Cl | -O- | 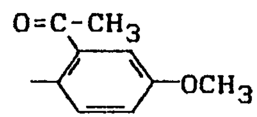 | 112 | analog Beispiel 2 |
| 254 | Cl | -O- | 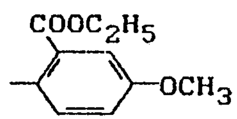 | 102 | analog Beispiel 2 |
| 255 | Cl | -O- | 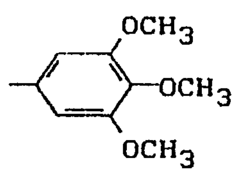 | 141 | analog Beispiel 2 |
| 256 | Cl | -O- | 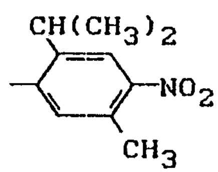 | 104 | analog Beispiel 2 |

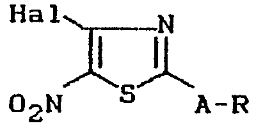

(I)

| Verbin-dungs-Nummer | Hal | - A - | - R | Schmp. (°C) | Herstellung |
|---|---|---|---|---|---|
| 257 | Cl | -O- | 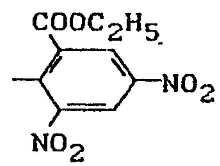 | 101 | analog Beispiel 2 |
| 258 | Cl | -O- | 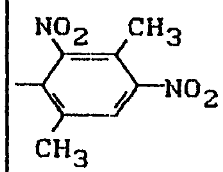 | 137 | analog Beispiel 2 |
| 259 | Cl | -O- | 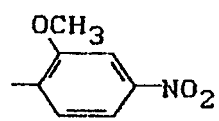 | 129-30 | analog Beispiel 2 |
| 260 | Cl | -O- | 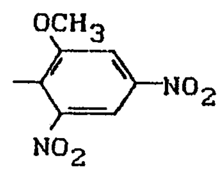 | 149 | analog Beispiel 2 |
| 261 | Cl | -O- | 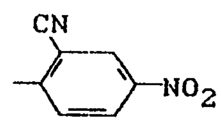 | 128-9 | analog Beispiel 2 |

Anwendungsbeispiele

In den folgenden Anwendungsbeispielen wurden die nachstehend aufgeführten Verbindungen als

Vergleichssubstanzen eingesetzt:

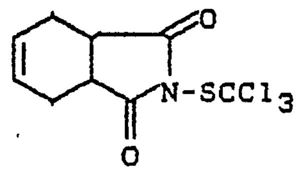

(A)

N-Trichlormethylthio-tetrahydrophthalimid (vergl. US 2.553.770) oder

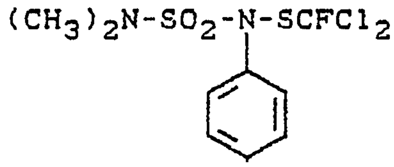

(B)

N,N-Dimethyl-N′-phenyl-N′-(fluordichlormethylthio)-sulfamid (vergl. DAS 1.193.498) oder

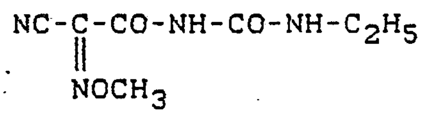

(C)

2-Cyan-N-(ethylaminocarbonyl)-2-methoximino)-acetamid (vergl. US 3.957.847) oder

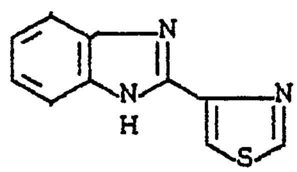

(D)

4-Benzimidazol-2-yl-thiazol (vgl. R. Wegler "Chemmie der Pflanzenschutz- und Schädlingsbekämpfungsmittel", Bd. 2, S. 124, Springer-Verlag, Berlin, Heidelberg, N.Y. 1970)

Beispiel A

Venturia-Test (Apfel) / protektiv

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung. Bei einer Wirkstoffkonzentration von beispielsweise 5 ppm zeigen viele der erfindungsgemäßen Verbindungen einen Wirkungsgrad zwischen 80 und 85 % gegenüber der unbehandelten Kontrolle.

Beispiel B

Plasmopara-Test (Reben) / protektiv

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Viele der erfindungsgemäßen Verbindungen zeigen bei einer Wirkstoffkonzentration von 10 ppm einen Wirkungsgrad zwischen 80 und 95 % gegenüber der unbehandelten Kontrolle.

Beispiel C

Phytophthora-Test (Tomate) /protektiv

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Bei einer Wirkstoffkonzentration von 10 ppm zeigen viele der erfindungsgemäßen Verbindungen einen Wirkungsgrad zwischen 80 und 95 % gegenüber der unbehandelten Kontrolle.

Beispiel D

Leptosphaeria nodorum-Test (Weizen) / protektiv

| | |
|---|---|
| Lösungsmittel: | 100 Gewichtsteile Dimethylformamid |
| Emulgator: | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Bei 0,025 Gew.-% in der Spritzbrühe zeigen einige der erfindungsgemäßen Verbindungen einen 100-%igen Wirkungsgrad gegen die unbehandelte Kontrolle.

## Beispiel E

Zum Nachweis der Wirksamkeit gegen Pilze bei technischen Materialien werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt.

So haben z.B. die Verbindungen Nr. 3, 6, 7, 9, 11, 45, 52, 53, 54, 55, 56, 64, 80, 77, 78 und 92, ebenso wie Beispiele A1 und A2 eine gute Wirkung und ein breites Wirkungsspektrum, z.B. bei den folgenden Testorganismen:

Alternaria tenuis
Aspergillus niger
Aureobasidium pullulans
Chaetomium globosum
Cladosporium cladosporioides
Lentinus tigrinus
Penicillium glaucum
Sclerophoma pityophila
Trichoderma viride

## Beispiel F

Wirkung gegen Bakterien bei technischen Materialien

Ein Agar, der als Nährmedium Bouillon enthält, wird mit erfindungsgemäßen Wirkstoffen in Konzentrationen von 1 bis 5000 ppm versetzt. Darauf infiziert man das Nährmedium jeweils mit den in der unten angegebenen Tabelle aufgeführten Testorganismen und hält das infizierte Medium 2 Wochen bei 28°C und 60 bis 70 % rel. Luftfeuchtigkeit. Die MHK ist die niedrigste Konzentration an Wirkstoff, bei der keinerlei Bewuchs durch die verwendete Mikrobenart erfolgt.

Tabelle

Escherichia coli
Staphylococcus aureus

Bei diesen Testorganismen zeigen z.B. die Verbindungen 3, 6, 9, 53, 77, 78 und Beispiele A1 und A2

eine bedeutend bessere Wirkung als die bekannte Vergleichssubstanz (D).

Beispiel G

Eine Mischkultur von Grün- Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phae odactylum tricornutum Bohlin) wird unter Durchperlen von Luft in Allens Nährlösung (Arch. Mikrobiol. 17, 34 bis 53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemäßer Wirkstoffe erkennt man an dem Entfärben der Nährlösung. So haben z.B. die Beispiele A1 und A2 eine gute Wirkung.

Tabelle H (Wirkung gegen Schleimorganismen)

Erfindungsgemäße Verbindungen werden in Konzentrationen von jeweils 0,1 bis 100 mg/l in Allens Nährlösung (Arch. Mikrobiol. 17, 34 bis 53 (1952), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

So zeigt z.B. die Verbindung von Beispiel A2 eine sehr gute Wirkung.

## Ansprüche

1. 4-Halogen-5-nitrothiazol-Derivate der Formel (I)

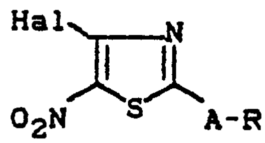

(I)

in welcher
Hal für Halogen steht,
A für O, S, SO, $SO_2$ oder N-$R^1$ steht,
worin
$R^1$ für Wasserstoff, Alkyl, Alkenyl, Halogenalkyl, Cyanalkyl oder für jeweils gegebenenfalls ein- bis mehrfach, gleich oder verschieden substituiertes Cycloalkyl, Aryl oder Aralkyl steht und
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl substituiertes Cycloalkyl steht, wobei
zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Alkinyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht oder
R und $R^1$ zusammen mit dem Stickstoffatom, an dem sie stehen, einen Ring bilden, der gegebenenfalls

durch ein oder mehrere weitere, gleiche oder verschiedene Heteroatome unterbrochen sein kann und der gegebenenfalls ein- bis mehrfach, gleich oder verschieden substituiert sein kann,
mit der Ausnahme, daß R nicht Wasserstoff ist, wenn A für SO oder $SO_2$ steht.

2. 4-Halogen-5-nitrothiazol-Derivate gemäß Anspruch 1, worin in der Formel (I)
Hal für Halogen steht und
A-R für OR steht, worin
R für Alkyl, Alkenyl oder Alkinyl steht, für gegebenenfalls ein- bis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkoxy, Halogenalkylmercapto, Alkylsulfonylamino, Alkylsulfonyl, Aryl, Aryloxy, Arylmercapto, Acyloxy, Acyl, Sulfamoyl, N-alkylsulfamoyl, N,N-Dialkylsulfamoyl, Aralkyloxy, Aralkylmercapto, Acylamino, Acylalkylamino, Cycloalkyl und Cyan substituiertes Aryl steht,
oder
A-R für SR steht, worin
R für Alkyl, Alkenyl oder Alkinyl steht, für gegebenenfalls ein- bis mehrfach, gleich oder verschieden durch Alkyl substituiertes Cycloalkyl steht, wobei zusätzlich zu der Alkyl-substitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkoxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht,
oder
A-R für $SO_nR$ steht, worin
n für 1 oder 2 steht und
R für gegebenenfalls ein- bis mehrfach, gleich oder verschieden durch Halogen, Alkyl, Alkenyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkoxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht,
oder
A-R für -NH-R steht, worin
R für Alkenyl, Alkinyl, für gegebenenfalls einbis mehrfach, gleich oder verschieden durch Alkyl substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Halogenalkyl, Nitro, Dialkylamino, Carbalkoxy, Carbamoyl, N-Alkylcarbamoyl, N,N-Dialkylcarbamoyl, Halo genmethyloxy, Halogenmethylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht,
oder
A-R für

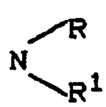

steht, worin
$R^1$ für Alkyl, Alkenyl, Halogenalkyl, Cyanalkyl oder für jeweils gegebenenfalls ein- bis mehrfach, gleich oder verschieden substituiertes Cycloalkyl, Aryl oder Aralkyl steht und
R für Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl

substituiertes Cycloalkyl steht, wobei zusätzlich zu der Alkylsubstitution ein Ring ankondensiert sein kann, für gegebenenfalls ein- bis mehrfach, gleich oder verschieden durch Halogen, Alkyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Alkenyl, Halogenalkyl, Nitro, Dialkylamino, Carbalkoxy, Carbamoyl, N-Alkylcarbamoyl, N,N-Dialkylcarbamoyl, Halogenmethyloxy, Halogenmethylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht oder

R und $R^1$ zusammen mit dem Stickstoffatom, an dem sie stehen, einen Ring bilden, der gegebenenfalls durch ein oder mehrere weitere, gleiche oder verschiedene Heteroatome unterbrochen sein kann und der gegebenenfalls ein- bis mehrfach, gleich oder verschieden substituiert sein kann.

3. 4-Halogen-5-nitrothiazol-Derivate gemäß Anspruch 1, worin in der Formel (I)

Hal für Fluor, Chlor, Brom oder Iod steht,

Patentansprüche

1. 4-Halogen-5-nitrothiazol-Derivate der Formel (I)

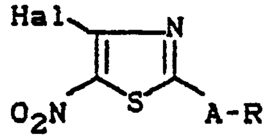

(I)

in welcher

Hal für Halogen steht,

A für O, S, SO, $SO_2$ oder N-$R^1$ steht,

worin

$R^1$ für Wasserstoff, Alkyl, Alkenyl, Halogenalkyl, Cyanalkyl oder für jeweils gegebenenfalls ein- bis mehrfach, gleich oder verschieden substituiertes Cycloalkyl, Aryl oder Aralkyl steht und

R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls ein- bis mehrfach, gleich oder verschieden durch Alkyl substituiertes Cycloalkyl steht, wobei

gleich oder verschieden durch Fluor, Chlor, Brom oder Alkyl mit 1 bis 4 Kohlenstoffatomen substituiert sein können, für gegebenenfalls ein- bis dreifach, gleich oder verschieden durch Alkyl mit 1 bis 4 Kohlenstoffatomen substituiertes Cycloalkyl mit 3 bis 8 Kohlenstoffatomen steht, wobei der Cycloalkylring zusätzlich einen ankondensierten Ring enthalten kann, für gegebenenfalls ein- bis fünffach, gleich oder verschieden durch Halogen, Alkyl mit 1 bis 4 Kohlenstoffatomen, Halogenalkyl mit 1 bis 4 Kohlenstoffatomen und 1 bis 8 gleichen oder verschiedenen Halogenatomen, Nitro, Cyano, Alkoxy mit 1 bis 4 Kohlenstoffatomen oder Alkylmercapto mit 1 bis 4 Kohlenstoffatomen substituiertes Phenylalkyl mit 1 bis 4 Kohlenstoffatomen im Alkylteil steht, wobei der Alkylteil gegebenenfalls einen weiteren Phenylrest als Substituenten enthält, der gegebenenfalls, wie vorher angegeben, substituiert sein kann, oder für gegebenenfalls ein- bis fünffach, gleich oder verschieden durch Halogen, Nitro, Alkyl mit 1 bis 12 Kohlenstoffatomen, Alkoxy, Alkylmercapto, Carbalkoxy, Alkylsulfonylamino, Alkylsulfonyl, Sulfamoyl, N-Alkylsulfamoyl, N,N-Dialkylsulfamoyl, Dialkylamino, Carbamoyl, N-Alkylcarbamoyl, N,N-Dialkylcarbamoyl mit jeweils 1 bis 4 Kohlenstoffatomen je Alkylrest, Alkenyl oder Alkinyl mit bis zu 6 Kohlenstoffatomen, Halogenalkyl, Halogenalkoxy, Halogenalkylmercapto mit jeweils 1 bis 4 Kohlenstoffatomen und 1 bis 8 gleichen oder verschiedenen Halogenatomen je aufgezähltem Rest, Phenyl, Phenoxy, Phenylmercapto, Acyloxy mit 1 bis 3 Kohlenstoffatomen, Acyl mit 1 bis 3 Kohlenstoffatomen, Phenylalkyloxy mit 1 bis 3 Kohlenstoffatomen im Alkylteil, Phenylalkylmercapto mit 1 bis 3 Kohlenstoffatomen, Acylamino mit 1 bis 3 Kohlenstoffatomen, Acylalkylamino mit 1 bis 3 Kohlenstoffatomen je Acyl- und Alkylrest und Cycloalkyl mit 4 bis 6 Kohlenstoffatomen und Cyan substituiertes Phenyl oder für Naphthyl steht oder

R und $R^1$ gemeinsam mit dem Stickstoffatom, an dem sie stehen, einen Ring mit 5 bis 7 Ringgliedern bilden, der gegebenenfalls ein oder zwei weitere Stickstoff- und/oder Sauerstoffatome enthalten kann und gegebenenfalls ein- bis dreifach durch Alkyl mit 1 bis 4 Kohlenstoffatomen substituiert sein kann,

mit der Ausnahme, daß R nicht Wasserstoff ist, wenn A für SO oder $SO_2$ steht.

4. 4-Halogen-5-nitrothiazol-Derivate gemäß Anspruch 1, worin in der Formel (I)

Hal für Chlor, Brom oder Iod steht,
A für O, S, SO, $SO_2$ oder $NR^1$ steht, wobei
$R^1$ für Wasserstoff, Alkyl mit 1 bis 4 Kohlenstoffatomen, Alkenyl mit 3 oder 4 Kohlenstoffatomen, für gegebenenfalls ein- bis dreifach durch Alkyl mit 1 bis 3 Kohlenstoffatomen substituiertes Phenyl, Benzyl oder 2-Phenyl-2-methylethyl steht und
R für Wasserstoff, Alkyl mit 1 bis 8 Kohlenstoffatomen, Alkenyl mit 3 bis 8 Kohlenstoffatomen, Alkinyl mit 4 bis 8 Kohlenstoffatomen, Cycloalkyl mit 3 bis 6 Kohlenstoffatomen, Cyanalkyl mit 1 bis 3 Kohlenstoffatomen im Alkylteil, oder für gegebenenfalls ein- bis fünffach, gleich oder verschieden durch Alkyl mit 1 bis 12 Kohlenstoffatomen, Alkenyl mit bis zu 3 Kohlenstoffatomen, Fluor, Chlor, Brom, Iod, Trifluormethyl, Nitro, Alkoxy mit 1 bis 4 Kohlenstoffatomen, Alkylmercapto mit 1 bis 4 Kohlenstoffatomen, Dialkylamino mit 1 oder 2 Kohlenstoffatomen je Alkylrest, Carbalkoxy mit 1 oder 2 Kohlenstoffatomen, Trifluormethoxy, Trifluormethylmercapto, Alkylsulfonylamino mit 1 oder 2 Kohlenstoffatomen, Alkylsulfonyl mit 1 oder 2 Kohlenstoffatomen, Phenyl, Phenoxy, Phenylmercapto, Acetoxy, Acetyl, Sulfamoyl, N-Alkylsulfamoyl mit 1 oder 2 Kohlenstoffatomen, N,N-Dialkylsulfamoyl mit 1 oder 2 Kohlenstoffatomen je Alkylrest, Carbamoyl, N-Alkylcarbamoyl mit 1 oder 2 Kohlenstoffatomen, N,N-Dialkylcarbamoyl mit 1 oder 2 Kohlenstoffatomen je Alkylrest, Benzyloxy, Benzylmercapto, Formylamino, For mylmethylamino, Acetylamino, Cyclopentyl, Cyclohexyl oder Cyano substituiertes Phenyl, Benzyl oder Phenethyl oder Naphthyl steht oder
R und $R^1$ gemeinsam mit dem Stickstoffatom, an dem sie stehen, für gegebenenfalls ein- oder zweifach durch Alkyl mit 1 oder 2 Kohlenstoffatomen substituiertes Pyrrolidin, Piperidin, Hexamethylenimin, Morpholin, N-Alkylpiperazin ($C_1$-$C_2$), Pyrrol, Pyrazol, Imidazol oder 1,2,4-Triazol stehen,
mit der Ausnahme, daß R nicht Wasserstoff ist, wenn A für SO oder $SO_2$ steht.

5. Verfahren zur Herstellung von 4-Halogen-5-nitrothiazol-Derivaten der Formel (I)

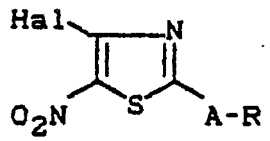

(I)

in welcher
Hal für Halogen steht,
A für O, S, SO, $SO_2$ oder N-$R^1$ steht,
worin
$R^1$ für Wasserstoff, Alkyl, Alkenyl, Halogenalkyl, Cyanalkyl oder für jeweils gegebe nenfalls ein- bis mehrfach, gleich oder verschieden substituiertes Cycloalkyl, Aryl oder Aralkyl steht und
R für Wasserstoff, Alkyl, Alkenyl oder Alkinyl steht, wobei die vorgenannten Reste jeweils gegebenenfalls ein- bis mehrfach, gleich oder verschieden durch Halogen, Alkoxy, Aryloxy, Alkylmercapto, Arylmercapto und Cyano substituiert sein können, für gegebenenfalls einbis mehrfach, gleich oder verschieden durch Alkyl substituiertes Cycloalkyl steht, wobei zusätzlich zu der Alkylsubstitution ein Ring ankondensiert sein kann, für gegebenenfalls ein-bis mehrfach, gleich oder verschieden durch Halogen, Alkyl, Alkenyl, Alkinyl, Halogenalkyl, Nitro, Alkoxy, Alkylmercapto und Cyan im Arylteil substituiertes Aralkyl steht oder für gegebenenfalls ein- bis mehrfach, gleich oder verschieden durch Halogen, Alkyl, Halogenalkyl, Nitro, Alkoxy, Alkylmercapto, Dialkylamino, Carbalkoxy, Carbamoyl, N-Alkylcarbamoyl, N,N-Dialkylcarbamoyl, Halogenalkyloxy, Halogenalkylmercapto, Alkylsulfonylamino, Alkylsulfonyl, Aryl, Aryloxy, Arylmercapto, Acyloxy, Acyl, Sulfamoyl, N-Alkylsulfamoyl, N,N-Dialkylsulfamoyl, Aralkyloxy, Aralkylmercapto, Acylamino, Acylalkylamino, Cycloalkyl und Cyan substituiertes Aryl steht oder
R und $R^1$ zusammen mit dem Stickstoffatom, an dem sie stehen, einen Ring bilden, der gegebenen falls durch ein oder mehrere weitere, gleiche oder verschiedene Heteroatome unterbrochen sein kann und der gegebenenfalls ein- bis mehrfach, gleich oder verschieden substituiert sein kann,
mit der Ausnahme, daß R nicht Wasserstoff ist, wenn A für SO oder $SO_2$ steht, dadurch gekennzeichnet, daß

a) für den Fall, daß A für O, S, $SO_2$ oder $NR^1$ steht,
2,4-Dihalogen-5-nitrothiazole der Formel (II)

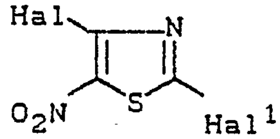

(II)

in welcher
Hal und $Hal^1$ gleiche oder verschiedene Halogenatome bedeuten,
mit Nucleophilen der Formel (III)
$H - A' - R$ (III)
in welcher
R die oben angegebene Bedeutung hat und
$A'$ für O, S, $SO_2$ oder $NR^1$ steht, wobei $R^1$ die vorher angegebene Bedeutung hat, oder deren Metallsalze gegebenenfalls in Gegenwart von Säurebindemitteln sowie in Gegenwart von Verdünnungsmitteln umsetzt, oder

b) für den Fall, daß AR für

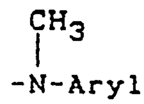

steht, 2,4-Dihalogen-5-nitrothiazole der Formel (II) mit N,N-Dimethylarylaminen der Formel (IV)

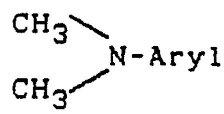 (IV)

in welcher Aryl die unter R für Aryl angegebene Bedeutung hat, in Gegenwart von Verdünnungsmitteln umgesetzt werden,
oder daß

c) für den Fall, daß A für $SO_2$ bzw. SO steht, 4-Halogen-5-nitrothiazolyl-sulfide der Formel (V)

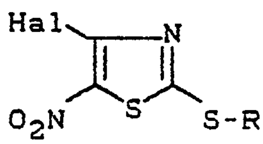 (V)

in welcher Hal und R die oben angegebene Bedeutung haben,
α) mit mindestens 2 Mol eines Oxidationsmittels in Gegenwart eines unter den Reaktionsbedingungen inerten Verdünnungsmittels bzw.
β) mit etwa 1 Mol eines Oxidationsmittels in Gegenwart eines unter den Reaktionsbedingungen inerten Verdünnungsmittels umgesetzt werden.

6. Schädlingsbekämpfungsmittel, gekennzeichnet durch einen Gehalt an mindestens einem 4-Halogen-5-nitrothiazol-Derivat der Formel (I) nach den Ansprüchen 1 oder 5.

7. Verwendung von 4-Halogen-5-nitrothiazol-Derivaten der Formel (I) nach den Ansprüchen 1 oder 5 zur Bekämpfung von Schädlingen.

8. Verwendung von 4-Halogen-5-nitrothiazol-Derivaten der Formel (I) gemäß Anspruch 7 zur Bekämpfung von Schädlingen im Pflanzenbereich und in technischen Materialien.

9. Verfahren zur Bekämpfung von Schädlingen, dadurch gekennzeichnet, daß man 4-Halogen-5-nitrothiazol-Derivate der Formel (I) nach den Ansprüchen 1 oder 5 auf Schädlinge und/oder ihren Lebensraum einwirken läßt.

10. Verfahren zur Herstellung von Schädlingsbekämpfungsmitteln, dadurch gekennzeichnet, daß man 4-Halogen-5-nitrothiazol-Derivate der Formel (I) nach den Ansprüchen 1 oder 5 mit Streckmitteln und/oder oberflächenaktiven Mitteln vermischt.

11. 2,4-Dihalogen-5-nitrothiazole der Formel (II A)

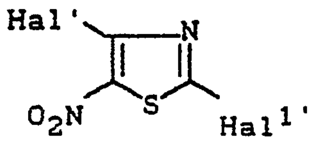

(II A)

in welcher ,

Hal′ und Hal¹ unabhängig voneinander für Chlor, Iod oder Fluor stehen, mit der Maßgabe, daß nicht beide Hals gleichzeitig Chlor sein dürfen.

12. Verfahren zur Herstellung von 2,4-Dihalogen-5-nitrothiazolen der Formel (II A)

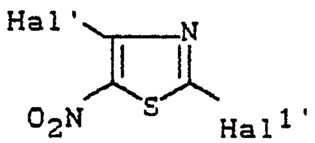

(II A)

in welcher ,

Hal′ und Hal¹ unabhängig voneinander für Chlor, Iod oder Fluor stehen, mit der Maßgabe, daß nicht beide Hals gleichzeitig Chlor sein dürfen,

dadurch gekennzeichnet, daß man 2,4-Dichlor-5-nitrothiazol mit Metalliodiden bzw. Metallfluoriden umsetzt.

13. Verwendung von 2,4-Dihalogen-5-nitrothiazolen der Formel (IIA) nach den Ansprüchen 11 und 12 zur Bekämpfung von Mikroben in technischen Materialien.